(19) Europäisches Patentamt
European Patent Office
Office européen des brevets

(11) EP 4 129 997 A1

(12) **EUROPEAN PATENT APPLICATION**
published in accordance with Art. 153(4) EPC

(43) Date of publication:
**08.02.2023 Bulletin 2023/06**

(21) Application number: **21781242.9**

(22) Date of filing: **06.04.2021**

(51) International Patent Classification (IPC):
***C07D 403/14*** *(1974.07)* ***C07D 413/14*** *(1974.07)*
***C07D 498/22*** *(1974.07)* ***A61K 31/551*** *(2000.01)*
***A61K 31/553*** *(2000.01)* ***A61P 35/00*** *(2000.01)*

(52) Cooperative Patent Classification (CPC):
**A61K 31/551; A61K 31/553; A61P 35/00; C07D 403/14; C07D 413/14; C07D 498/22**

(86) International application number:
**PCT/CN2021/085733**

(87) International publication number:
**WO 2021/197499 (07.10.2021 Gazette 2021/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.04.2020 CN 202010260612**
**19.06.2020 CN 202010568775**
**30.12.2020 CN 202011616152**

(71) Applicant: **Medshine Discovery Inc.**
**Nanjing, Jiangsu 210032 (CN)**

(72) Inventors:
- **CHEN, Shuhui**
  **Shanghai 200131 (CN)**
- **CHEN, Kevin X**
  **Shanghai 200131 (CN)**
- **ZHOU, Kai**
  **Shanghai 200131 (CN)**
- **JIANG, Wen**
  **Shanghai 200131 (CN)**
- **HU, Guoping**
  **Shanghai 200131 (CN)**
- **LI, Jian**
  **Shanghai 200131 (CN)**

(74) Representative: **Dehns**
**St. Bride's House**
**10 Salisbury Square**
**London EC4Y 8JD (GB)**

(54) **OCTAHYDROPYRAZINODIAZANAPHTHYRIDINE DIONE COMPOUNDS**

(57) Disclosed is a class of octahydropyrazinodiazanaphthyridine dione compounds, and specifically disclosed are a compound as shown in formula (III) and a pharmaceutically acceptable salt thereof.

EP 4 129 997 A1

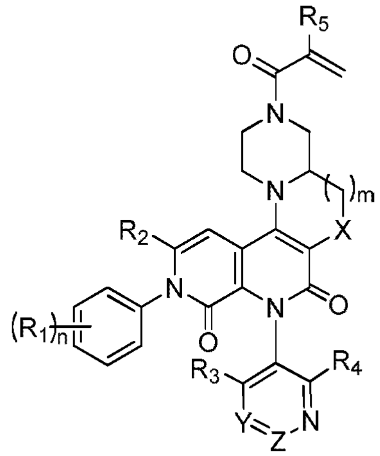

(III)

**Description**

The present application claims the following priorities:

CN202010260612.X, filed on April 03, 2020;
CN202010568775.4, filed on June 19, 2020;
CN202011616152.6, filed on December 30, 2020.

TECHNICAL FIELD

The present disclosure relates to a new class of octahydropyrazinodiazanaphthyridine dione compounds, in particular to a compound represented by formula (III) and a pharmaceutically acceptable salt thereof.

BACKGROUND

The first RAS oncogene was found in rat sarcoma, hence the name. RAS protein is the product expressed by the RAS gene, which refers to a class of closely related monomeric globulins composed of 189 amino acids with a molecular weight of 21 KDa. It can bind to a guanine trinucleotide phosphate (GTP) or a guanine dinucleotide phosphate (GDP). The active state of the RAS protein has effects on cell growth, differentiation, cytoskeleton, protein transport and secretion, etc., and its activity is regulated by binding to GTP or GDP. When the RAS protein binds to GDP, it is in a dormant state, that is, in an "inactivated" state; when stimulated by specific upstream cell growth factors, the RAS protein is induced to exchange GDP and bind to GTP, which is called "activated" state. The RAS protein bound to GTP can activate downstream proteins for signal transmission. The RAS protein itself has weak hydrolysis activity to hydrolyze GTP and can hydrolyze GTP to GDP. In this way, the transformation from the activated state to the inactivated state can be realized. In this hydrolysis process, GAP (GTPase activating proteins) is also required. It can interact with RAS protein, greatly promoting its ability to hydrolyze GTP to GDP. Mutation of the RAS protein will affect its interaction with GAP, which also affects its ability to hydrolyze GTP to GDP, leaving the RAS protein always in an activated state. The activated RAS protein continuously gives downstream proteins growth signals, which eventually leads to continuous growth and differentiation of cells, and ultimately produce tumors. There are many members of RAS gene family, among which subfamilies closely related to various cancers mainly include Kirsten rat sarcoma viral oncogene homolog (KRAS), Harvey rat sarcoma viral oncogene homolog (HRAS) and neuroblastoma rat sarcoma viral oncogene homolog (NRAS). It has been found that about 30 % of human tumors carry some mutated RAS gene, among which KRAS mutation is the most significant, accounting for 86 % of all RAS mutations. For KRAS mutations, the most common mutations are found at residues of glycine at position 12 (G12), glycine at position 13 (G13) and glutamine at position 61 (Q61), of which G12 mutation accounts for 83 %.

The G12C mutation is one of the more common mutations in the KRAS gene, which refers to the mutation of glycine to cysteine at position 12. KRAS G12C mutation is the most common in lung cancer. According to the data reported in the literature, KRAS G12C mutation accounts for about 10 % of all lung cancer patients.

CONTENT OF THE PRESENT INVENTION

The present disclosure provides a compound represented by formula (III) or a pharmaceutically acceptable salt thereof,

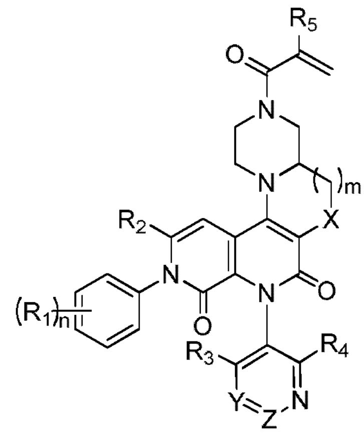

(III)

wherein,

m is selected from 0, 1 and 2;
n is selected from 0, 1, 2, 3 and 4;
X is selected from NH and O;
Y is selected from CH and N;
Z is selected from CH and N;
$R_1$ is each independently H, F, Cl, Br, I, OH, $NH_2$ and $C_{1-3}$ alkyl, and the $C_{1-3}$ alkyl is optionally substituted by 1, 2, or 3 $R_a$;
$R_2$ is selected from H, F, Cl, Br, I and $C_{1-3}$ alkyl, and the $C_{1-3}$ alkyl is optionally substituted by 1, 2, or 3 $R_b$;
$R_3$ is selected from H, F, Cl, Br, I and $C_{1-6}$ alkyl, and the $C_{1-6}$ alkyl is optionally substituted by 1, 2, or 3 $R_c$;
$R_4$ is selected from H, F, Cl, Br, I and $C_{1-6}$ alkyl, and the $C_{1-6}$ alkyl is optionally substituted by 1, 2, or 3 $R_c$;
$R_5$ is selected from H, F, Cl, Br and I;
$R_a$, $R_b$ and $R_c$ are each independently selected from H, F, Cl, Br and I.

In some embodiments of the present disclosure, the compound has a structure of formula (II):

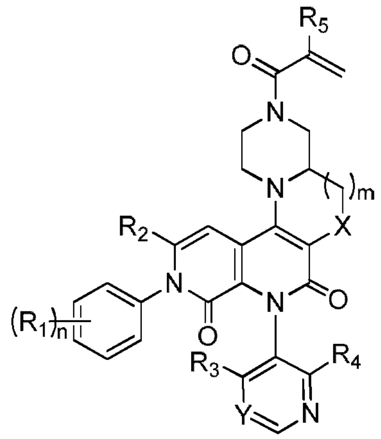

(II)

,

$R_1$, $R_2$, $R_3$, $R_4$, $R_5$, X, Y, m and n are as defined in the present disclosure.

In some embodiments of the present disclosure, the $R_1$ is each independently selected from F, Cl, $NH_2$ and OH, and other variables are as defined in the present disclosure.

In some embodiments of the present disclosure, the $R_2$ is selected from $CH_3$, and the $CH_3$ is optionally substituted by 1, 2 or 3 $R_b$, and other variables are as defined in the present disclosure.

In some embodiments of the present disclosure, the $R_2$ is selected from $CF_3$, and other variables are as defined in the present disclosure.

In some embodiments of the present disclosure, the $R_3$ is selected from $CH_3$, $CH_2CH_3$ and

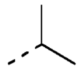,

and other variables are as defined in the present disclosure.

In some embodiments of the present disclosure, the $R_4$ is selected from $CH_3$, $CH_2CH_3$ and

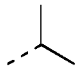,

and other variables are as defined in the present disclosure.

In some embodiments of the present disclosure, the $R_5$ is selected from Hand F, and other variables are as defined in the present disclosure.

In some embodiments of the present disclosure, the structural moiety

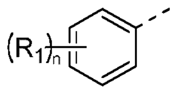

is selected from

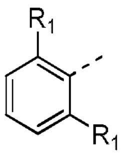 and 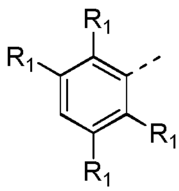;

, and other variables are as defined in the present disclosure.

In some embodiments of the present disclosure, the structural moiety

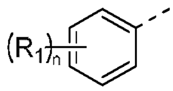

is selected from

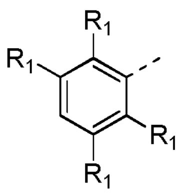,

and other variables are as defined in the present disclosure.

In some embodiments of the present disclosure, the structural moiety

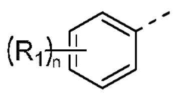

is selected from

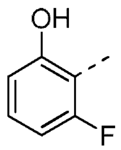 and 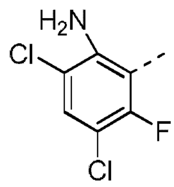 ,

and other variables are as defined in the present disclosure.

In some embodiments of the present disclosure, the structural moiety

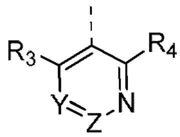

is selected from

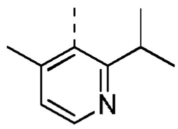 , 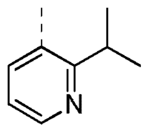 , 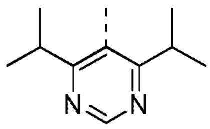 , 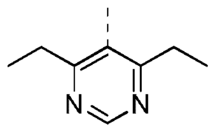

and

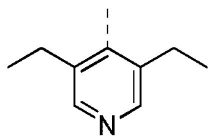 ,

and other variables are as defined in the present disclosure.

In some embodiments of the present disclosure, the structural moiety

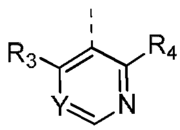

is selected from

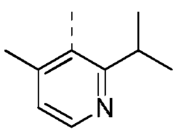 , 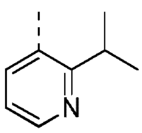 , 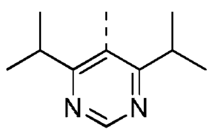 and 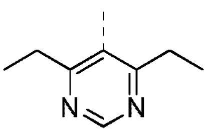 ;

, and other variables are as defined in the present disclosure.

In some embodiments of the present disclosure, the compound is selected from

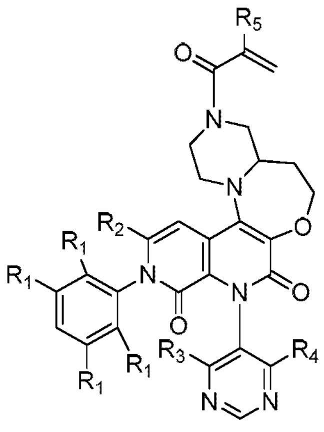
(II-2) ,

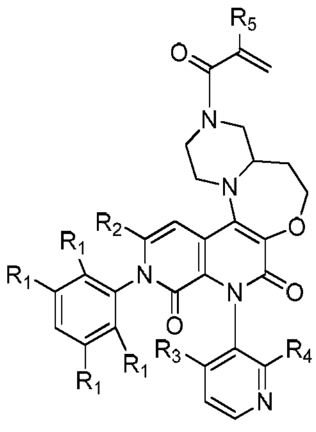
(II-3) and

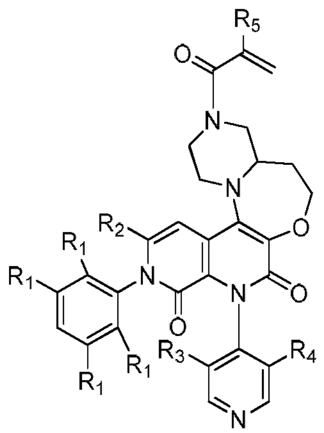
(III-1) ,

wherein, $R_1$, $R_2$, $R_3$, $R_4$ and $R_5$ are as defined in the present disclosure.

In some embodiments of the present disclosure, the compound or the pharmaceutically acceptable salt thereof, wherein the compound is selected from

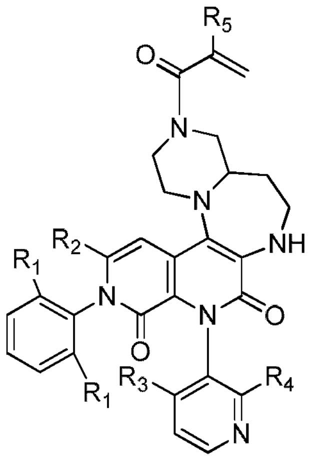
(II-1) ,

wherein, $R_1$, $R_2$, $R_3$, $R_4$ and $R_5$ are as defined in the present disclosure.

The present disclosure provides a compound represented by the following formula or a pharmaceutically acceptable salt thereof, selected from:

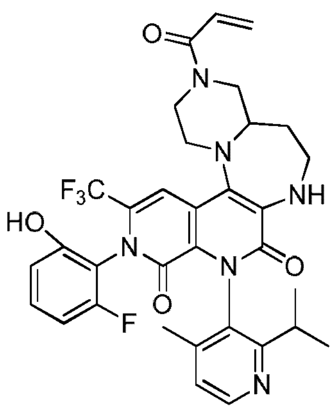
,

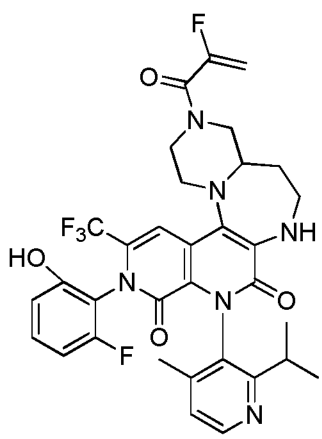
,

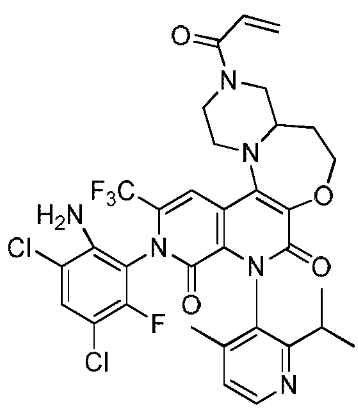
,

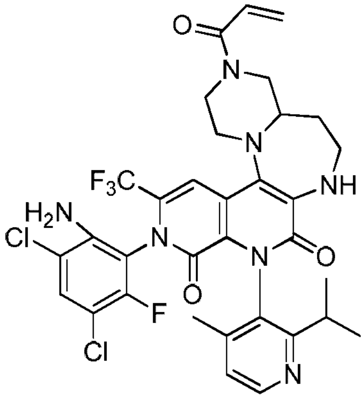,
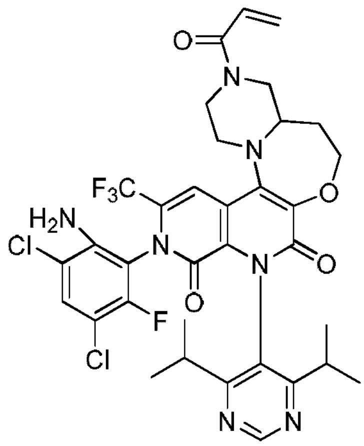,
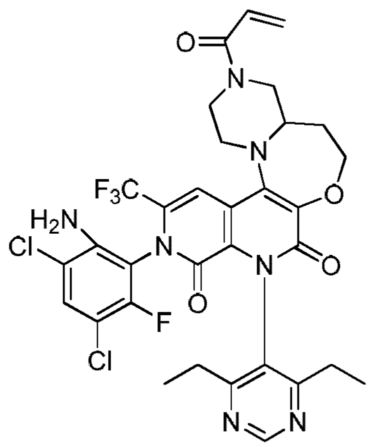

and

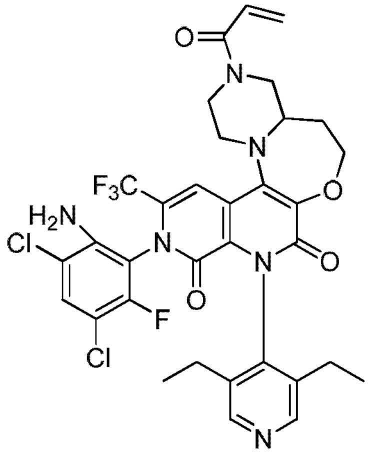.

In some embodiments of the present disclosure, the compound or the pharmaceutically acceptable salt thereof is selected from:

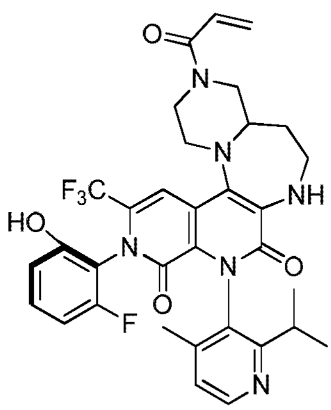,
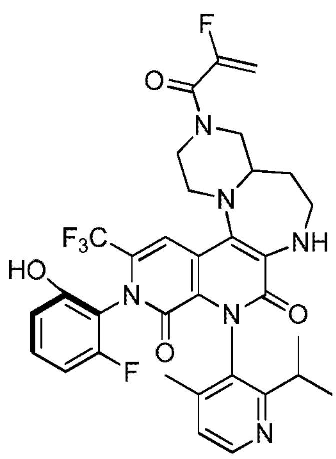,
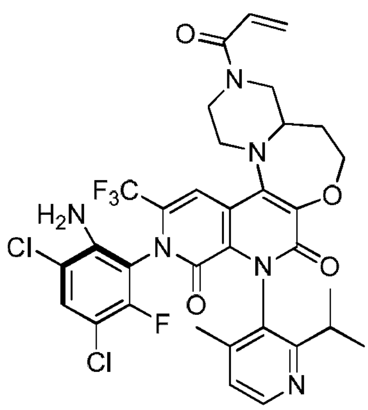,

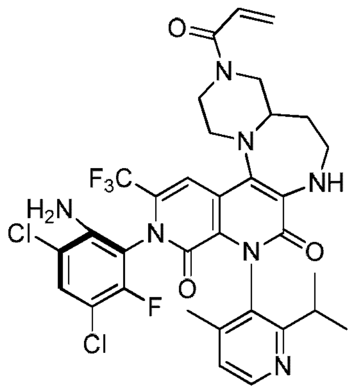,
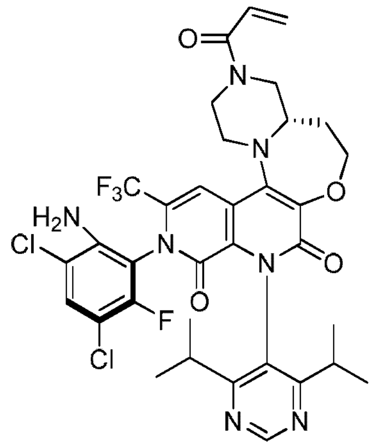,
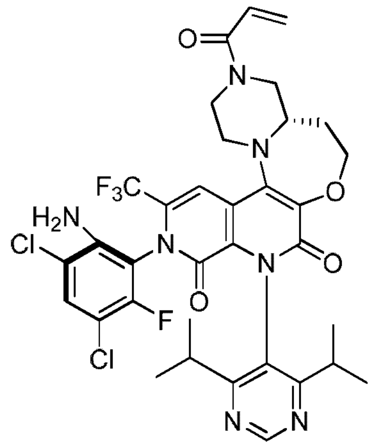,

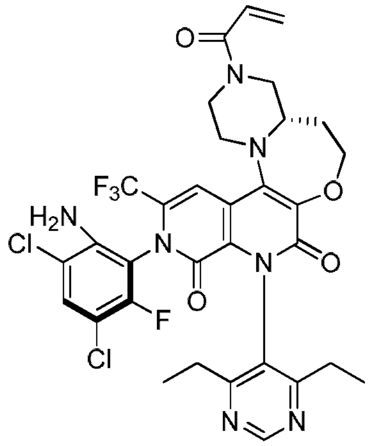,
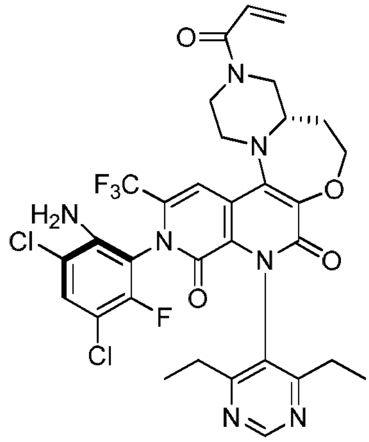,
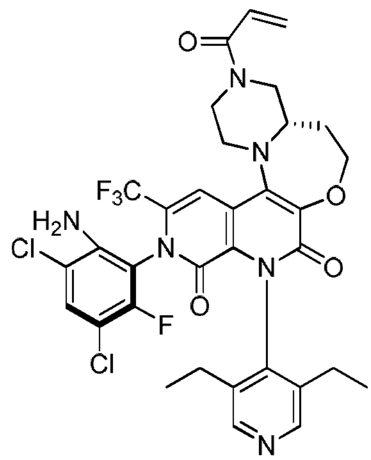

and

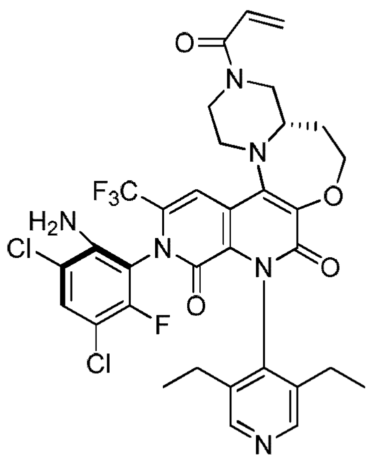.

There are also some embodiments of the present disclosure which are any combination of the above variables.

The present disclosure provides a pharmaceutical composition, comprising a therapeutically effective amount of the compound or the pharmaceutically acceptable salt thereof described in the present disclosure as an active ingredient and a pharmaceutically acceptable carrier.

The present disclosure provides a use of the above compound or the pharmaceutically acceptable salt thereof or the above composition in the manufacture of a KRAS G12C mutant protein inhibitor.

The present disclosure provides a use of the above compound or the pharmaceutically acceptable salt thereof or the above composition in the manufacture of a medicament for the treatment of cancer.

**Definition and description**

Unless otherwise specified, the following terms and phrases when used herein have the following meanings. A specific term or phrase should not be considered indefinite or unclear in the absence of a particular definition, but should be understood in the ordinary sense. When a trading name appears herein, it is intended to refer to its corresponding commodity or active ingredient thereof. The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, an allergic reaction or other problems or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt may be obtained by bringing the neutral form of the compound into contact with a sufficient amount of base in a pure solution or a suitable inert solvent. The pharmaceutically acceptable base addition salt includes a salt of sodium, potassium, calcium, ammonium, organic amine or magnesium, or similar salts. When the compound of the present disclosure contains a relatively basic functional group, an acid addition salt may be obtained by bringing the neutral form of the compound into contact with a sufficient amount of acid in a pure solution or a suitable inert solvent. Examples of the pharmaceutically acceptable acid addition salt include an inorganic acid salt, wherein the inorganic acid includes, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid, and the like; and an organic acid salt, wherein the organic acid includes, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid, and the like; and salts of amino acid (such as arginine and the like), and a salt of an organic acid such as glucuronic acid and the like. Certain specific compounds of the present disclosure contain both basic and acidic functional groups, thus can be converted to any base or acid addition salt.

The pharmaceutically acceptable salt of the present disclosure can be prepared from the parent compound that contains an acidic or basic moiety by conventional chemical method. Generally, such salt can be prepared by reacting the free acid or base form of the compound with a stoichiometric amount of an appropriate base or acid in water or an organic solvent or a mixture thereof.

In addition to salt forms, the compounds provided in the present disclosure also exist in prodrug forms. The prodrugs of the compounds described herein easily undergo chemical changes under physiological conditions to be converted into the compounds of the present disclosure. Furthermore, the prodrugs may be converted to the compounds of the present disclosure by chemical or biochemical methods in an *in vivo* environment.

Some compounds of the present disclosure may exist in a non-solvated or solvated form, including a hydrate form. Generally speaking, the solvated form is equivalent to the non-solvated form, and both are included in the scope of the present disclosure.

The compounds of the present disclosure may exist in specific geometric or stereoisomeric forms. The present disclosure contemplates all such compounds, including *cis* and *trans* isomers, (-)- and (+)-enantiomers, (*R*)- and (*S*)-enantiomers, diastereomers isomers, (*D*)-isomers, (*L*)-isomers, and racemic and other mixtures thereof, such as enantiomers or diastereomeric enriched mixtures, all of which are within the scope of the present disclosure. Additional asymmetric carbon atoms may be present in substituents such as alkyl. All these isomers and their mixtures are included within the scope of the present disclosure.

Unless otherwise specified, the term "enantiomer" or "optical isomer" refers to stereoisomers that are mirror images of each other.

Unless otherwise specified, the term "*cis-trans* isomer" or "geometric isomer" is caused by the inability to rotate freely of double bonds or single bonds of ring-forming carbon atoms.

Unless otherwise specified, the term "diastereomer" refers to a stereoisomer in which a molecule has two or more chiral centers and the relationship between the molecules is not mirror images.

Unless otherwise specified, "(*D*)" or "(+)" refers to dextrorotation, "(*L*)" or "(-)" refers to levorotation, and "(*DL*)" or "(±)" refers to racemic.

Unless otherwise specified, the absolute configuration of a stereogenic center is represented by a wedged solid bond ( 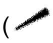 ) and a wedged dashed bond ( 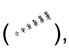 ), and the relative configuration of a stereogenic center is represented by a straight solid bond ( 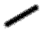 ) and a straight dashed bond ( 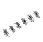 ), a wave line ( 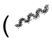 ) is used to represent a wedged solid bond ( 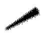 ) or a wedged dashed bond ( 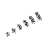 ), or the wave line ( 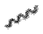 ) is used to represent a straight solid bond ( 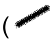 )

and a straight dashed bond (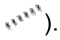).

The compounds of the present disclosure may exist in specific. Unless otherwise specified, the term "tautomer" or "tautomeric form" means that at room temperature, the isomers of different functional groups are in dynamic equilibrium and can be transformed into each other quickly. If tautomers possibly exist (such as in solution), the chemical equilibrium of tautomers can be reached. For example, proton tautomer (also called prototropic tautomer) includes interconversion through proton migration, such as keto-enol isomerization and imine-enamine isomerization. Valence tautomer includes some recombination of bonding electrons for mutual transformation. A specific example of keto-enol tautomerization is the tautomerism between two tautomers of pentane-2,4-dione and 4-hydroxypent-3-en-2-one.

Unless otherwise specified, the terms "enriched in one isomer", "enriched in isomers", "enriched in one enantiomer" or "enriched in enantiomers" refer to the content of one of the isomers or enantiomers is less than 100 %, and the content of the isomer or enantiomer is greater than or equal to 60 %, or greater than or equal to 70 %, or greater than or equal to 80 %, or greater than or equal to 90 %, or greater than or equal to 95 %, or greater than or equal to 96 %, or greater than or equal to 97 %, or greater than or equal to 98 %, or greater than or equal to 99 %, or greater than or equal to 99.5 %, or greater than or equal to 99.6 %, or greater than or equal to 99.7 %, or greater than or equal to 99.8 %, or greater than or equal to 99.9 %.

Unless otherwise specified, the term "isomer excess" or "enantiomeric excess" refers to the difference between the relative percentages of two isomers or two enantiomers. For example, if the content of one isomer or enantiomer is 90 %, and the content of the other isomer or enantiomer is 10 %, the isomer or enantiomer excess (ee value) is 80 %.

Optically active (*R*)- and (*S*)-isomers, as well as *D* and *L* isomer can be prepared using chiral synthesis or chiral reagents or other conventional techniques. If one kind of enantiomer of certain compound of the present disclosure is to be obtained, the pure desired enantiomer can be obtained by asymmetric synthesis or derivative action of chiral auxiliary followed by separating the resulting diastereomeric mixture and cleaving the auxiliary group. Alternatively, when the molecule contains a basic functional group (such as amino) or an acidic functional group (such as carboxyl), the compound reacts with an appropriate optically active acid or base to form a salt of the diastereomeric isomer which is then subjected to diastereomeric resolution through the conventional method in the art to obtian the pure enantiomer. In addition, the enantiomer and the diastereoisomer are generally isolated through chromatography which uses a chiral stationary phase and optionally combines with a chemical derivative method (such as carbamate generated from amine). The compound of the present disclosure may contain an unnatural proportion of atomic isotope at one or more than one atom(s) that constitute the compound. For example, the compound can be radiolabeled with a radioactive isotope, such as tritium ($^{3}H$), iodine-125 ($^{125}I$) or C-14 ($^{14}C$). For another example, deuterated drugs can be formed by replacing hydrogen with heavy hydrogen, the bond formed by deuterium and carbon is stronger than that of ordinary hydrogen and carbon, compared with non-deuterated drugs, deuterated drugs have the advantages of reduced toxic and side effects, increased drug stability, enhanced efficacy, extended biological half-life of drugs and the like. All isotopic variations of the compound of the present disclosure, whether radioactive or not, are encompassed within the scope of the present disclosure. The term "optional" or "optionally" means that the subsequent event or condition may occur but not requisite, that the term includes the instance in which the event or condition occurs and the instance in which the event or condition does not occur.

The term "substituted" means one or more than one hydrogen atom(s) on a specific atom are substituted with the substituent, including deuterium and hydrogen variables, as long as the valence of the specific atom is normal and the substituted compound is stable. When the substituent is an oxygen (i.e., =O), it means two hydrogen atoms are substituted. Positions on an aromatic ring cannot be substituted with a ketone. The term "optionally substituted" means an atom can be substituted with a substituent or not, unless otherwise specified, the type and number of the substituent may be arbitrary as long as is chemically achievable.

When any variable (such as R) occurs in the constitution or structure of the compound more than once, the definition of the variable at each occurrence is independent. Thus, for example, if a group is substituted with 0-2 R, the group can be optionally substituted with up to two R, wherein the definition of R at each occurrence is independent. Moreover, a combination of the substituent and/or the variant thereof is allowed only when the combination results in a stable compound.

When the number of a linking group is 0, such as $-(CRR)_0-$, it means that the linking group is a single bond.

When one of the variables is selected from a single bond, it means that the two groups linked by the single bond are connected directly. For example, when L in A-L-Z represents a single bond, the structure of A-L-Z is actually A-Z.

When a substituent is vacant, it means that the substituent does not exist, for example, when X is vacant in A-X, the structure of A-X is actually A. When the enumerative substituent does not indicate by which atom it is linked to the group to be substituted, such substituent can be bonded by any atom thereof. For example, when pyridyl acts as a substituent, it can be linked to the group to be substituted by any carbon atom on the pyridine ring.

When the enumerative linking group does not indicate the direction for linking, the direction for linking is arbitrary, for example, the linking group L contained in

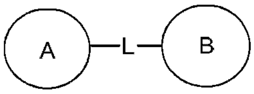

is -M-W-, then -M-W- may link ring A and ring B to form

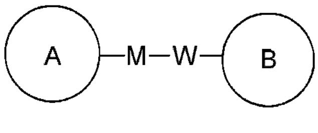

in the direction same as left-to-right reading order, and form

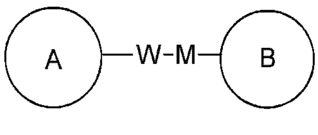

in the direction contrary to left-to-right reading order. A combination of the linking groups, substituents and/or variables thereof is allowed only when such combination can result in a stable compound.

Unless otherwise specified, the term "$C_{1-6}$ alkyl" refers to a linear or branched saturated hydrocarbon group consisting of 1 to 6 carbon atoms. The $C_{1-6}$ alkyl includes $C_{1-5}$, $C_{1-4}$, $C_{1-3}$, $C_{1-2}$, $C_{2-6}$, $C_{2-4}$, $C_6$, $C_5$ alkyl and the like; it may be monovalent (such as methyl), divalent (such as methylene) or multivalent (such as methine). Examples of $C_{1-6}$ alkyl include but are not limited to methyl (Me), ethyl (Et), propyl (including n-propyl and isopropyl), butyl (including n-butyl, isobutyl, s-butyl, and t-butyl), pentyl (including n-pentyl, isopentyl and neopentyl), hexyl and the like.

Unless otherwise specified, the term "$C_{1-3}$ alkyl" refers to a linear or branched saturated hydrocarbon group consisting of 1 to 3 carbon atoms. The $C_{1-3}$ alkyl includes $C_{1-2}$ and $C_{2-3}$ alkyl and the like; it may be monovalent (such as methyl), divalent (such as methylene) or multivalent (such as methine). Examples of $C_{1-3}$ alkyl include but are not limited to methyl (Me), ethyl (Et), propyl (including n-propyl and isopropyl) and the like.

Unless otherwise specified, $C_{n-n+m}$ or $C_n$-$C_{n+m}$ includes any specific case of n to n+m carbons, for example, $C_{1-12}$ includes $C_1$, $C_2$, $C_3$, $C_4$, $C_5$, $C_6$, $C_7$, $C_8$, $C_9$, $C_{10}$, $C_{11}$ and $C_{12}$, and any range from n to n+m is also included, for example $C_{1-12}$ includes $C_{1-3}$, $C_{1-6}$, $C_{1-9}$, $C_{3-6}$, $C_{3-9}$, $C_{3-12}$, $C_{6-9}$, $C_{6-12}$, $C_{9-12}$ and the like; similarly, n-membered to n+m-membered means that the number of atoms on the ring is from n to n+m, for example, 3-12-membered ring includes 3-membered ring, 4-membered ring, 5-membered ring, 6-membered ring, 7-membered ring, 8-membered ring, 9-membered ring, 10-membered ring, 11-membered ring, and 12-membered ring, and any range from n to n+m is also included, for example, 3-12-membered ring includes 3-6-membered ring, 3-9-membered ring, 5-6-membered ring, 5-7-membered ring, 6-7-membered ring, 6-8-membered ring, and 6-10-membered ring and the like.

The term "leaving group" refers to a functional group or atom that can be replaced by another functional group or atom by a substitution reaction (such as an affinity substitution reaction). For example, representative leaving groups include triflate; chlorine, bromine, and iodine; sulfonate group, such as mesylate, tosylate, *p*-bromobenzenesulfonate, *p*-toluenesulfonates and the like; acyloxy, such as acetoxy, trifluoroacetoxy and the like.

The term "protecting group" includes, but is not limited to "amino protecting group", "hydroxy protecting group" or "thio protecting group". The term "amino protecting group" refers to a protecting group suitable for blocking the side reaction on the nitrogen of an amino. Representative amino protecting groups include, but are not limited to: formyl; acyl, such as alkanoyl (e.g., acetyl, trichloroacetyl or trifluoroacetyl); alkoxycarbonyl, such as *tert*-butoxycarbonyl (Boc); arylmethoxycarbonyl such as benzyloxycarbonyl (Cbz) and 9-fluorenylmethoxycarbonyl (Fmoc); arylmethyl, such as benzyl (Bn), trityl (Tr), 1,1-bis-(4'-methoxyphenyl)methyl; silyl, such as trimethylsilyl (TMS) and tert-butyldimethylsilyl (TBS) and the like. The term "hydroxy protecting group" refers to a protecting group suitable for blocking the side reaction on hydroxy. Representative hydroxy protecting groups include, but are not limited to: alkyl, such as methyl, ethyl, and *tert*-butyl; acyl, such as chain alkanoyl (e.g., acetyl); arylmethyl, such as benzyl (Bn), *p*-methoxybenzyl (PMB), 9-fluorenylmethyl (Fm), and diphenylmethyl (benzhydryl, DPM); silyl, such as trimethylsilyl (TMS) and *tert*-butyl dimethyl silyl (TBS) and the like.

The structure of the compounds of the present disclosure can be confirmed by conventional methods known to those skilled in the art, and if the disclosure involves an absolute configuration of a compound, then the absolute configuration can be confirmed by means of conventional techniques in the art. For example, in the case of single crystal X-ray diffraction (SXRD), the absolute configuration can be confirmed by collecting diffraction intensity data from the cultured single crystal using a Bruker D8 venture diffractometer with CuKα radiation as the light source and scanning

mode: φ/ω scan, and after collecting the relevant data, the crystal structure can be further analyzed by direct method (Shelxs97), so that the absolute configuration can be confirmed.

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred implementations include but are not limited to the embodiments of the present disclosure.

The solvent used in the present disclosure is commercially available.

The compounds of the present disclosure are named according to the conventional naming principles in the art or by ChemDraw® software, and the commercially available compounds use the supplier catalog names.

**Technical effect**

The compounds of the present disclosure are potent KRAS G12C mutant protein inhibitors.

BRIEF DESCRIPTION OF THE DRAWINGS

Figs. 1 and 2 show the protein expression of ERK and p-ERK in human colon cancer CO-04-0070 tumor tissue after administration.

DETAILED DESCRIPTION OF THE PREFERRED EMBODIMENT

The present disclosure is described in detail by the embodiments below, but it does not mean that there are any adverse restrictions on the present disclosure. The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred implementations include but are not limited to the embodiments of the present disclosure. It will be apparent to those skilled in the art that various variations and improvements can be made to specific embodiments of the present disclosure without departing from the spirit and scope of the present disclosure.

**Embodiment 1**

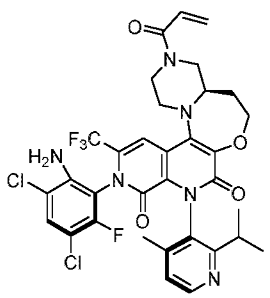

1A or 1B or 1C or 1D

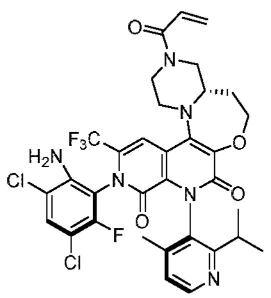

1A or 1B or 1C or 1D

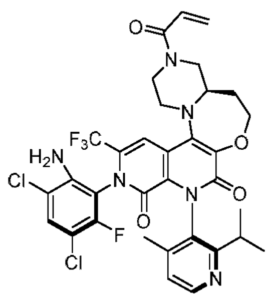

1A or 1B or 1C or 1D

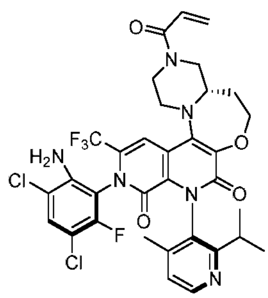

1A or 1B or 1C or 1D

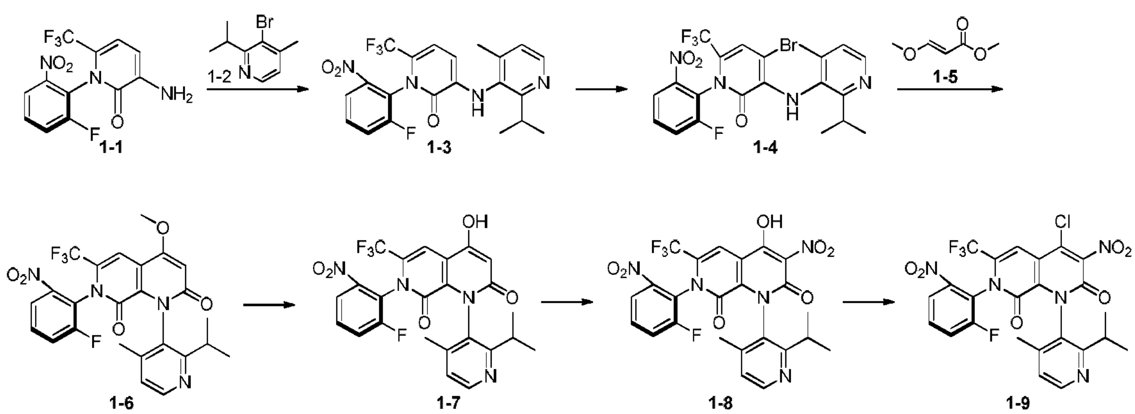

1-1 1-2 1-3 1-4 1-5

1-6 1-7 1-8 1-9

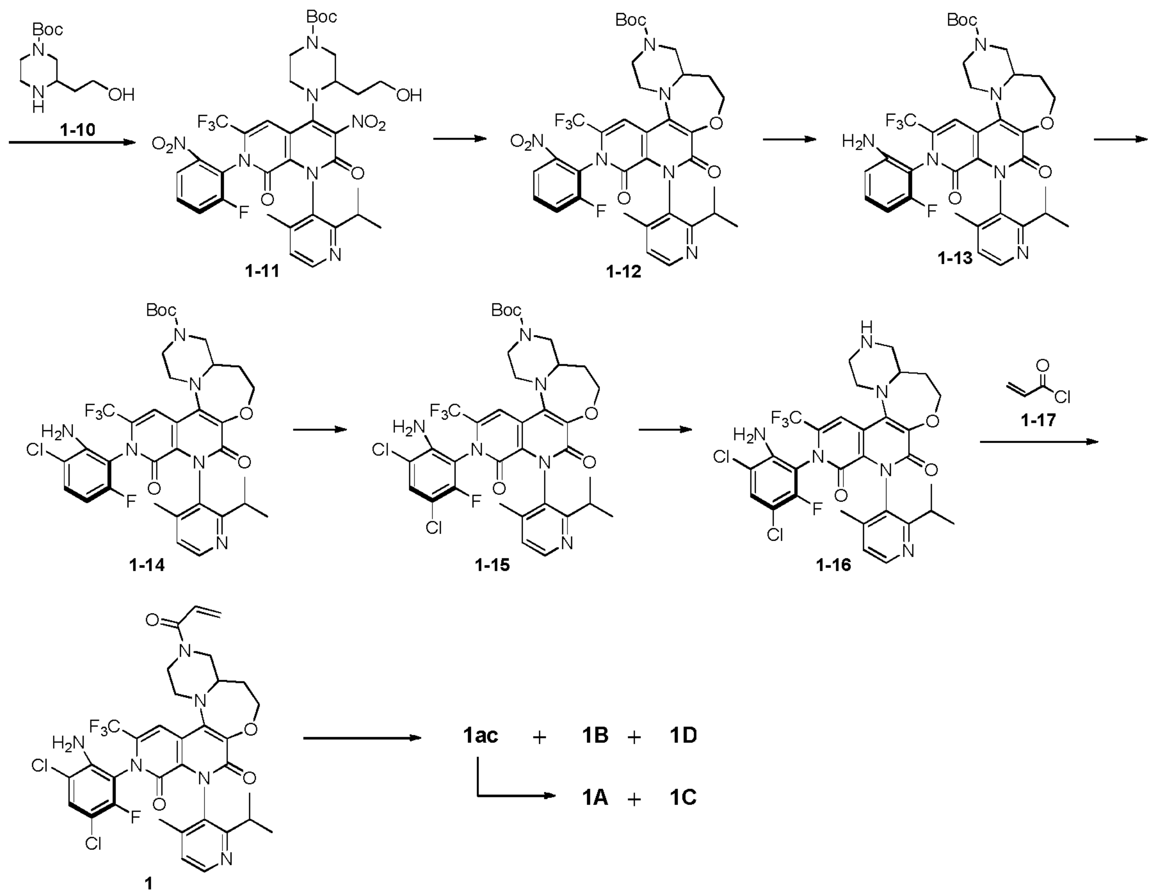


**Synthesis of 1-3:** BrettPhos-Pd-G3 (2.96 g, 3.27 mmol) and cesium carbonate (30.44 g, 93.41 mmol) were added to a solution of **compound 1-1** (14.82 g, 46.71 mmol) and **compound 1-2** (10 g, 46.71 mmol) in *tert*-pentanol (100 mL) at one time under nitrogen protection and stirred at 105 °C for 16 hours. The reaction solution was concentrated and then separated by silica gel column chromatography (petroleum ether: ethyl acetate of 10:1 to 5:1 (v/v)) to obtain **compound** 1-3. LCMS (ESI) m/z: 451 (M+1).

**Synthesis of 1-4:** N-Bromosuccinimide (4.90 g, 27.53 mmol) was added to a solution of **compound 1-3** (12.4 g, 27.53 mmol) in dichloromethane (120 mL) in batches at 0 °C under nitrogen protection, and stirred at 0 °C for 0.5 hours. The reaction solution was quenched with a saturated sodium sulfite solution (30 mL) and extracted with dichloromethane (30 mL, once). The combined organic phase was washed with saturated brine (10 mL, once), dried over sodium sulfate, filtered, and concentrated to obtain a crude product. The crude product was slurried with (petroleum ether: ethyl acetate of 10:1 (v/v), 50 mL) to obtain **compound 1-4.**

**Synthesis of 1-6:** Chloro[(tri-*tert*-butylphosphine)-2-(2-aminobiphenyl)]palladium(II) (561.50 mg, 1.10 mmol) and *N,N*-dicyclohexylmethylamine (1.18 g, 6.03 mmol) were added to a mixed solution of **compound 1-4** (2.90 g, 5.48 mmol) and **compound 1-5** (784.37 mg, 6.03 mmol) in toluene (40 mL) under nitrogen protection. The reaction solution was stirred at 100 °C for 16 hours. The reaction solution was concentrated and then separated by silica gel column chromatography (petroleum ether: ethyl acetate of 5:1 to 1:1 (v/v)) to obtain

**compound 1-6.**

**Synthesis of 1-7: Compound 1-6** (8 g, 15.03 mmol) was added to aqueous hydrochloric acid solution (12 mol/L, 150 mL) under nitrogen protection. The reaction solution was stirred at 100 °C for 16 hours. After the reaction was completed, the pH was adjusted to 4 with sodium bicarbonate, and then the mixture was extracted with dichloromethane (150 mL, 2 times). The organic phase was dried over sodium sulfate, filtered, and concentrated to obtain **compound 1-7.** LCMS (ESI) m/z: 519 (M+1).

**Synthesis of 1-8: Compound 1-7** (5 g, 9.64 mmol) was added to a mixed solution of acetic acid (30 mL) and fuming nitric acid (3.04 g, 48.22 mmol) at 0 °C, then sodium nitrite (3.33 g, 48.22 mmol) was added thereto, and the mixture was stirred at 20 °C for 0.5 hours. The reaction solution was diluted with ethyl acetate (150 mL), and then washed with saturated brine (50 mL, 3 times). The pH of the obtained organic phase was adjusted to 10 with saturated aqueous

sodium bicarbonate solution, and the organic phase was dried over anhydrous sodium sulfate, filtered, and concentrated to obtain **compound 1-8.** LCMS (ESI) m/z: 564 (M+1).

**Synthesis of 1-9:** Phosphorus oxychloride (4.14 mL, 44.55 mmol) was added to a solution of **compound 1-8** (5.02 g, 8.91 mmol) in acetonitrile (12 mL) at room temperature under nitrogen protection and stirred at 70 °C for 15 minutes. The reaction solution was concentrated, dissolved in acetonitrile (12 mL), concentrated once again, dried under reduced pressure by an oil pump for 15 minutes, dissolved in ethyl acetate (150 mL), washed with cold water (0 to 10 °C, 50 mL*3) and saturated brine (50 mL), dried over sodium sulfate, filtered, and concentrated to obtain a crude product of **compound 1-9.** LCMS (ESI) m/z: 582.0 (M+1).

**Synthesis of 1-11:** Under nitrogen protection, a 4A molecular sieve (10 g) was added to a solution of **compound 1-9** (4.00 g, 6.87 mmol) and **compound 1-10** (4.75 g, 20.62 mmol) in acetonitrile (60 mL), and the reaction solution was stirred at room temperature for 14 hours. TLC showed that the reaction was completed. The reaction solution was concentrated and then dissolved in ethyl acetate (300 mL), washed with water (50 mL×2), and the organic phase was concentrated, and the residue was separated by silica gel column chromatography (petroleum ether: ethyl acetate of 3:1 to 1:1 (v/v)) to obtain **compound 1-11.**

**Synthesis of 1-12:** 1,8-Diazabicyclo[5.4.0]undec-7-ene (0.79 g, 5.10 mmol) was added to a solution of **compound 1-11** (2.0 g, 2.58 mmol) in dry DMF (20 mL) under nitrogen protection. The reaction solution was stirred at 130 °C for 30 minutes. LCMS showed that the reaction was completed. The reaction solution was diluted with ethyl acetate (350 mL), washed with water (50 mL×3) and saturated brine (50 mL), concentrated, and the residue was separated by silica gel column chromatography (petroleum ether: ethyl acetate of 3:1 to 1:1 (v/v)) to obtain **compound 1-12.** LCMS (ESI) m/z: 729.26 (M+1).

**Synthesis of 1-13: Compound 1-12** (1.4 g, 1.92 mmol) was added to a mixed solution of ethanol (30 mL) and water (10 mL), and then ammonium chloride (1.03 g, 19.2 mmol) and iron powder (1.07 g, 19.2 mmol) were added to the above mixed solution and stirred at 80 °C for 2 hours. The reaction solution was filtered, and the filtrate was diluted with water (20 mL) and extracted with dichloromethane (50 mL, once). The organic phase was washed with saturated brine (10 mL, once), dried over sodium sulfate, filtered, and the filtrate was concentrated to obtain **compound 1-13.** LCMS (ESI) m/z: 699.2 (M+1).

**Synthesis of 1-14: Compound 1-13** (0.716 g, 1.02 mmol) was dissolved in acetic acid (10 mL) and acetonitrile (5 mL), then cooled to 0 to 5 °C. DCDMH (0.232 g, 1.18 mmol) was added to the above mixed solution and stirred for 25 minutes. The reaction solution was diluted with ethyl acetate (150 mL), washed with saturated sodium bisulfite (50 mL×2) and saturated sodium bicarbonate (50 mL), concentrated, and the residue was separated by silica gel column chromatography (petroleum ether: ethyl acetate of 1:1 to 0:1 (v/v)) to obtain **compound 1-14.**

**Synthesis of 1-15: Compound 1-14** (0.70 g, 0.95 mmol) was dissolved in acetic acid (7 mL) and acetonitrile (3.5 mL), then cooled to 0 to 5 °C. DCDMH (0.216 g, 1.02 mmol) was added to the above mixed solution and stirred for 30 minutes. The reaction solution was diluted with ethyl acetate (150 mL), washed with saturated sodium bisulfite (50 mL × 2) and saturated sodium bicarbonate (50 mL), concentrated, and separated by preparative HPLC (chromatographic column: Phenomenex luna C18 250 * 50 mm *10 μm; mobile phase: [water (0.225 % formic acid)-acetonitrile]; acetonitrile %: 35 %-70 %, 25 minutes) to obtain **compound 1-15.**

**Synthesis of 1-16: Compound 1-15** (138 mg, 0.18 mmol) was dissolved in dichloromethane (2.0 mL), cooled to 0 to 5 °C, and trifluoroacetic acid (1.5 mL) was added thereto and stirred for 40 minutes. The reaction solution was added with ethyl acetate (100 mL), washed with saturated sodium bicarbonate (40 mL×2) and saturated brine (40 mL), dried over anhydrous sodium sulfate, filtered and concentrated to obtain **compound 1-16.**

**Synthesis of 1A to 1D**: Triethylamine (36.38 mg, 0.36 mmol) and acryloyl chloride (17.00, 0.188 mmol) were added to a solution of **compound 1-16** (120 mg, 0.18 mmol) in dichloromethane (2 mL) under nitrogen protection and stirred at 0 °C for 30 minutes. The reaction solution was quenched with water (10 mL) and extracted with ethyl acetate (50 mL×2). The combined organic phases were washed with saturated brine (10 mL), dried over sodium sulfate, filtered and concentrated to obtain a crude product of compound 1. The crude product was separated by SFC (column information: REGIS (R,R) WHELK-O1 (250 mm*25 mm, 10 μm); mobile phase: [0.1 % ammonia water-methanol]; methanol %: 40 %-40 %, 3; 50 minutes) to obtain **1ac** (3.205-4.163 minutes), **compound 1D** (4.7 minutes), **compound 1B** (5.62 minutes). The **1ac** was separated by SFC again (column information: (s,s) WHELK-O1 (250 mm*50 mm, 10 μm); mobile phase: [0.1 % ammonia water-isopropanol]; isopropanol %: 60 %-60 %, 5.5 minutes; 90 minutes) to obtain **compound 1C** (6.175 minutes) and **compound 1A** (7.905 minutes).

**Compound 1A:** LCMS (ESI) m/z: 721.0 (M+1). **$^1$HNMR** (400 MHz, $CDCl_3$) δ = 8.38 (br, 1H), 7.31(d, *J*=6.8 Hz, 1H), 7.17(s, 1H), 6.93 (m, 1H), 6.35 (d, *J*= 16.8 Hz, 1H), 5.75 (d, *J=10.4 Hz,* 1H), 4.62 (br, 2H), 4.30-4.14 (br, 3H), 3.88 (br, 2H), 3.58 - 3.43 (m, 3H), 3.42-3.40 (m, 2H), 2.50 (br, 1H), 2.25 (br, 2H), 2.04 (s, 3H), 1.18-1.14 (m, 6H).

**Compound 1B:** LCMS (ESI) m/z: 721.2 (M+1). **$^1$HNMR** (400 MHz, $CDCl_3$) δ = 8.48 (d, *J=5.2 Hz,* 1H), 7.32(d, *J=6.8 Hz,* 1H), 7.17(s, 1H), 6.93 (m, 1H), 6.32 (d, *J=16.8 Hz,* 1H), 5.76 (dd, *J=10.4, 1.6Hz,* 1H), 4.55-4.95 (br, 2H), 4.25-4.10 (m, 3H), 3.84 (br, 2H), 3.60 - 3.40 (m, 3H), 3.00-3.25 (m, 2H), 2.75 (br, 1H), 2.24 (br, 2H), 2.04 (s, 3H), 1.18-1.14 (m, 6H).

**Compound 1C:** LCMS (ESI) m/z: 721.0 (M+1). **$^1$HNMR** (400 MHz, $CDCl_3$) $\delta$ =8.48 (d, *J=4.8 Hz,* 1H), 7.31(d, *J=6.8 Hz,* 1H), 7.14 (s, 1H), 7.02 (d, *J=4.4 Hz,* 1H), 6.52 (br, 1H) 6.36 (d, *J=16.8 Hz,* 1H), 5.75 (d, *J=10.4 Hz,* 1H), 4.55-4.80 (m, 2H), 4.07-4.14 (m, 3H), 3.85 (br, 2H), 3.60 (br, 1H), 3.45 (br, 1H), 2.95-3.20 (br, 2H), 2.54 (m, 1H), 2.25 (br, 2H), 2.00 (s, 3H), 1.18-1.14 (m, 6H).

**Compound 1D**: LCMS (ESI) m/z: 721.1 (M+1). **$^1$HNMR** (400 MHz, $CDCl_3$) $\delta$ = 8.48 (d, *J=5.2 Hz,* 1H), 7.31(d, *J=6.8 Hz,* 1H), 7.14 (s, 2H), 6.50 (m, 1H), 6.36 (d, *J=18.8 Hz,* 1H), 5.75 (d, *J=11.2 Hz,* 1H), 4.62-4.80 (br, 2H), 4.07-4.14 (m, 2H), 3.50-3.85 (m, 3H), 3.20-3.60 (m, 4H), 2.50 (br, 1H), 2.25 (br, 2H), 2.04 (s, 3H), 1.18-1.14 (m, 6H).

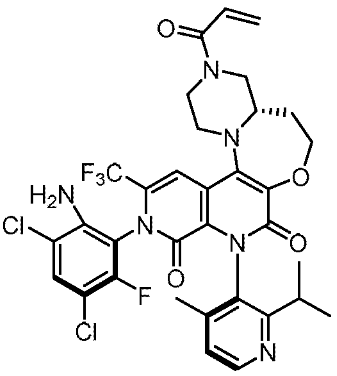

or

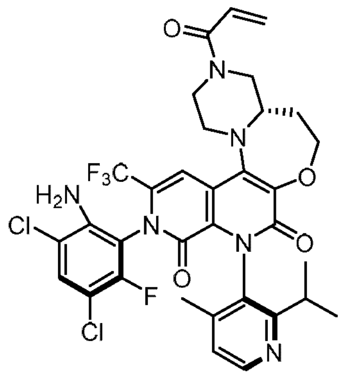

1D

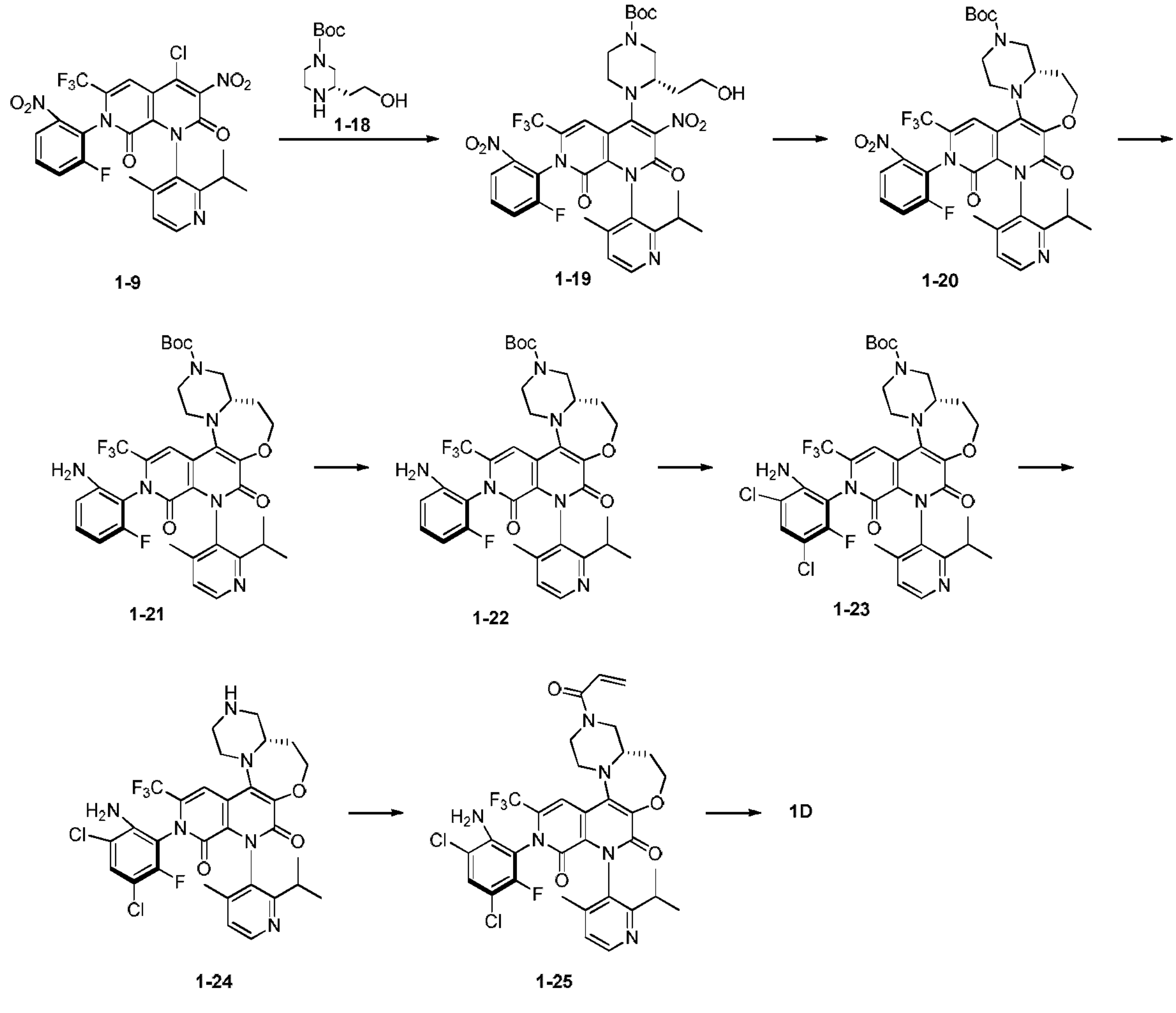


In the same way, the chiral piperazine compound 1-18 was used in the synthesis of step 1-11, and compound

1-25 was obtained through the same synthesis steps and method. Compound 1-25 was prepared by chiral SFC (column information: (s,s) WHELK-O1 (250 mm*50 mm, 10 μm); mobile phase: mobile phase A was supercritical carbon dioxide, mobile phase B was [0.1 % ammonia water-isopropanol]; elution gradient: 40 %-40 %) to obtain compound 1D, which was then prepared by HPLC (column information: Phenomenex Luna C8 250*50 mm*10 μm; mobile phase: mobile phase A was water containing 0.225 % formic acid, mobile phase B was acetonitrile; elution gradient: 20 %-60 % in 30 minutes) to obtain compound 1D.

Compound **1D**: LCMS (ESI) m/z: 721.2 (M+1). **$^1$HNMR** (400 MHz, $CDCl_3$) δ = 8.52 (d, *J*=5.02 Hz, 1 H),7.39 (d, *J*=7.2 Hz, 1 H), 7.23 (s, 1 H), 7.09 (br d, *J*=4.0 Hz, 1 H), 6.59 (br s, 1 H), 6.43 (dd, *J*=16.8, 1.6 Hz, 1 H), 5.84 (br d, *J*=10.4 Hz, 1 H) ,4.96 (br s, 1 H), 4.73 (br s, 1 H), 4.21 (br s, 1 H), 3.85 - 4.05 (m, 4 H), 3.43 - 3.73 (m, 2 H), 3.04 - 3.34 (m, 2 H), 2.58 (m, 1 H), 2.32 (br s, 2 H), 2.06 (s, 3 H), 1.21 (d, *J*=6.8 Hz, 3 H) 1.15 (d, *J*=6.8 Hz, 3 H).

SFC: ee value was 100 % and retention time was 2.951 minutes. (Column information: (S,S)Whelk-O1 100×4.6 mm I.D., 5.0 μm; mobile phase: mobile phase A was supercritical carbon dioxide, mobile phase B was [acetonitrile (containing 0.05 % dimethylamine)-isopropanol (V:V=1:2)]; elution gradient: 40 %, mobile phase B in supercritical carbon dioxide, flow rate: 2.5 mL/min, detector: PDA, column temperature: 40 °C, back pressure: 100 Bar.

**Embodiment 2**

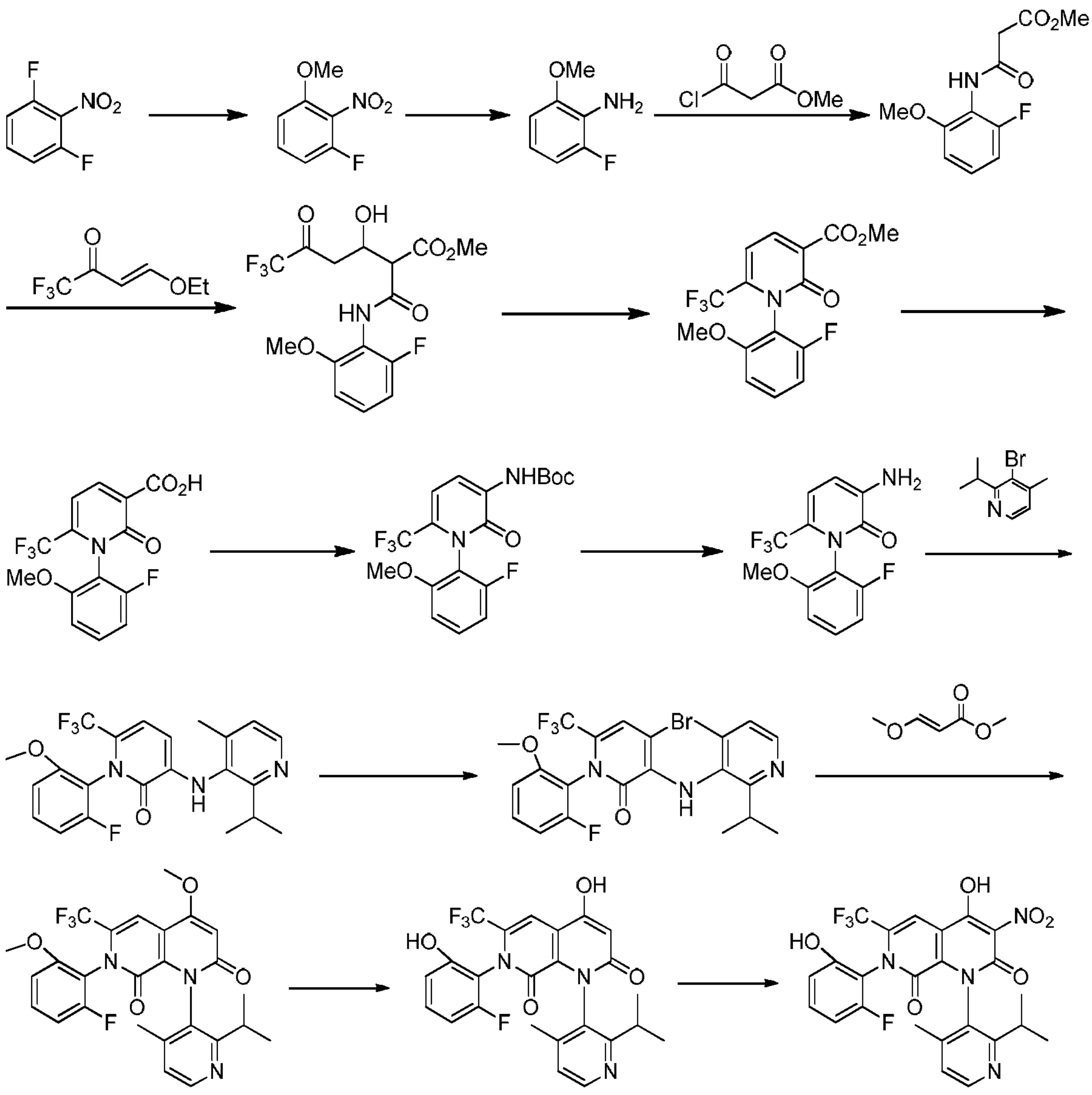

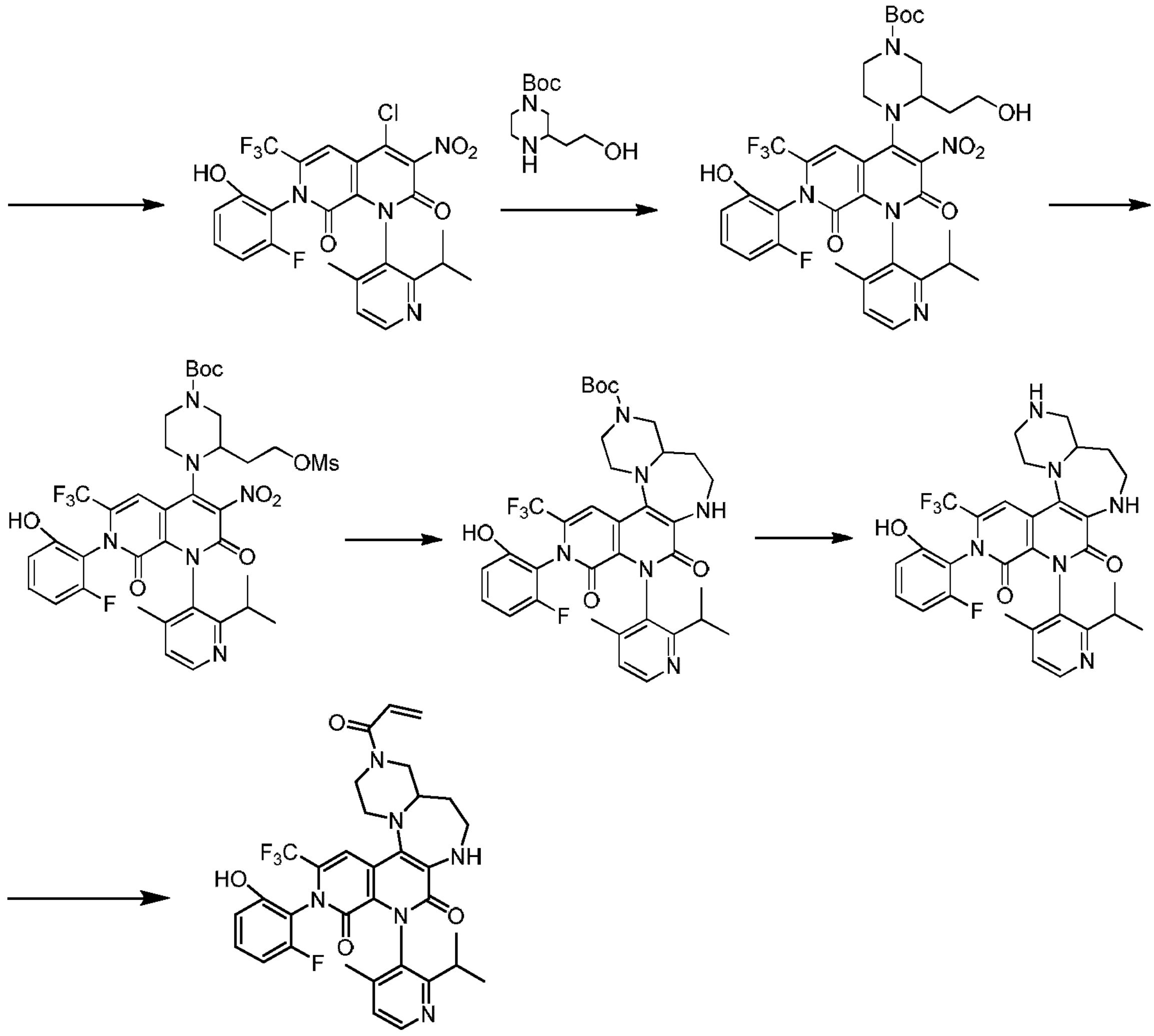

Compound 2

**Embodiment 3**

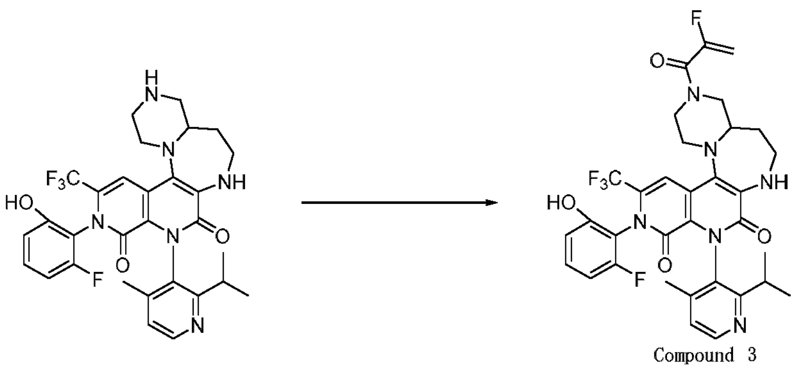

Compound 3

**Embodiment 4**

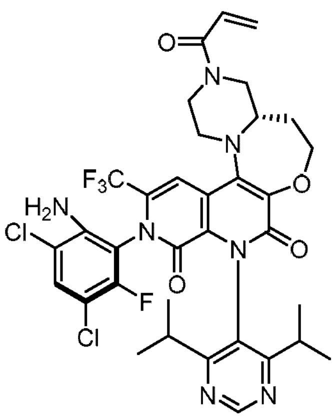
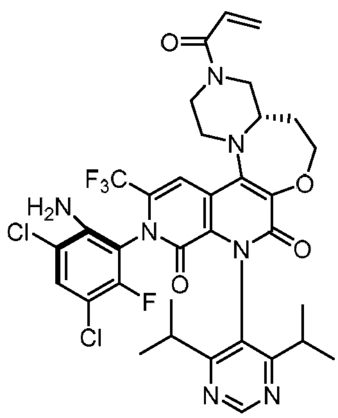

4A or 4B　　　　4B or 4A

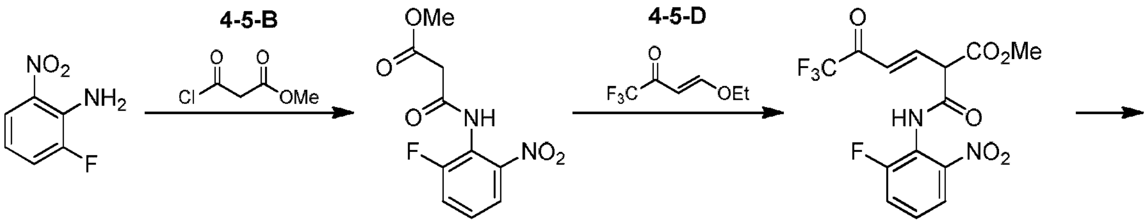


4-5-A　　　4-5-C　　　4-5-E

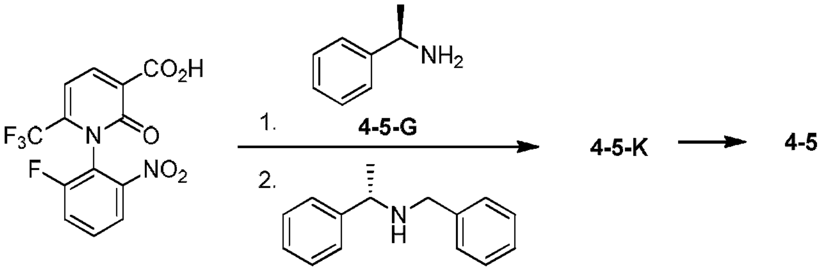


4-5-F　　　4-5-H

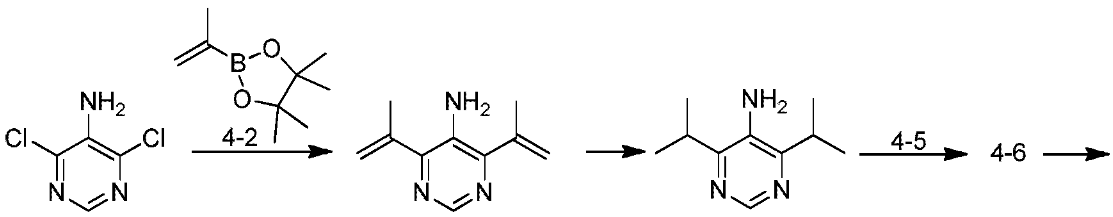


4-1　　　4-3　　　4-4

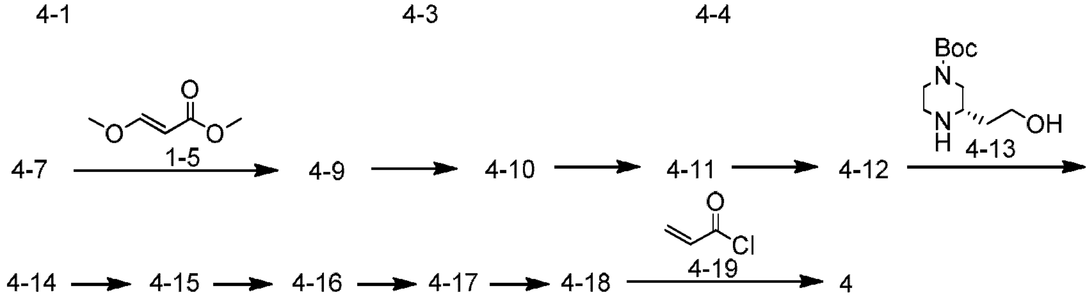


## Synthesis of 4-5-C:

At 0 °C, methyl malonyl chloride (51.20 g, 374.97 mmol, 40.00 mL, 1.17 *eq)* was slowly added dropwise to a suspension of compound **4-5-A** (50 g, 320.28 mmol, 1 *eq),* potassium carbonate (55.00 g, 397.93 mmol, 1.24 *eq)* and

acetonitrile (500 mL). After the dropwise addition was completed, the mixture was stirred at 20 °C for 16 hours. The reaction solution was filtered with diatomite, and the filter cake was washed with ethyl acetate (100 mL*2), and the crude product obtained by concentrating the filtrate was slurried with petroleum ether/ethyl acetate (V/V=3/1; 150 mL/50 mL) for 16 hours, and then filtered. The filter cake was washed with the above slurrying solvent (50 mL*2), and the solid was collected and dried to obtain compound **4-5-C.** The relevant characterization data were as follows: LCMS m/z: 256.9 $[M+1]^+$; $^1$H NMR (400MHz, $CDCl_3$) $\delta$ = 9.92 (br s, 1H), 7.84 (td, J=1.2, 8.4Hz, 1H), 7.49 - 7.41 (m, 1H), 7.40 - 7.32 (m, 1H), 3.85 (s, 3H), 3.57 (s, 2H).

**Synthesis of 4-5-E:**

Cesium carbonate (78 g, 239.40 mmol, 1.02 *eq)* was added to a solution of compound **4-5-C** (60 g, 234.20 mmol, 1 *eq)* in acetonitrile (600 mL), and then compound (*E*)-4-ethoxy-1,1,1-trifluorobut-3-en-2-one (40.12 g, 238.65 mmol, 34 mL, 1.02 *eq)* was added dropwise to the reaction solution. The reaction solution was stirred at 20 °C for 1 hour, and filtered after the reaction was completed. The filter cake was washed with ethyl acetate (100 mL*2), and then the organic phases were combined and concentrated to obtain a crude product. The crude product was slurried with petroleum ether/ethyl acetate (V/V=10/1, 600 mL/60 mL) for 1 hour and then filtered. The obtained filter cake was washed with the above slurrying solvent (50 mL*3), and the filter cake was collected and dried under vacuum to obtain compound **4-5-E.** The relevant characterization data were as follows: LCMS m/z: 379.0 $[M+1]^+$; $^1$H NMR (400MHz, DMSO-$d_6$) $\delta$ = 11.40 (br s, 1H), 8.67 - 8.31 (m, 1H), 7.81 - 7.56 (m, 2H), 7.34 (dt, J=5.5, 8.3 Hz, 1H), 7.01 - 6.02 (m, 1H), 3.83 - 3.63 (m, 3H).

**Synthesis of 4-5-F:**

Triethylamine (50.89 g, 502.92 mmol, 70 mL, 1.47 *eq)* was added to a solution of compound **4-5-E** (129 g, 341.06 mmol, 1 *eq)* in 2,2,2-trifluoroethanol (650 mL). The reaction solution was reacted at 80 °C for 16 hours. After the reaction was completed, the reaction solution was concentrated to obtain a residue. 500 mL of ethyl acetate was added to the residue, stirred for 1 hour, and then filtered, and the filter cake was washed with ethyl acetate (50 mL*2). The filter cake was collected and dried under vacuum to obtain compound **4-5-F.** The relevant characterization data were as follows: LCMS m/z: 346.9 $[M+1]^+$; $^1$H NMR (400MHz, DMSO-$d_6$) $\delta$ = 8.25 (d, J=8.3 Hz, 1H), 8.07 - 7.85 (m, 2H), 7.37 (d, J=8.0 Hz, 1H), 6.99 (d, J=8.0 Hz, 1H).

**Synthesis of 4-5-K:**

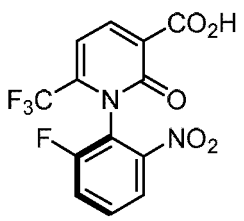

or

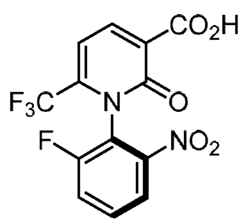

**4-5-K**

A solution of **4-5-G** (15.75 g, 0.5 *eq)* in isopropyl acetate (90 mL) was added to a suspension of compound **4-5-F** (90 g) in isopropyl acetate (810 mL) at 25 °C to obtain a clear solution. A seed crystal (30 mg, ee value of 91 %) was added, and the mixture was stirred at 25 °C for 16 hours. A large amount of solid was precipitated, filtered, and the mother liquor was washed with 2N hydrochloric acid. When **4-5-G** was completely removed, the mother liquor was concentrated to give a residue. The residue was then added with acetonitrile (540 mL) and a solution of **4-5-H** (29.1 g, 0.8 *eq.)* in acetonitrile (20 mL) to obtain a clear solution. After 5 minutes, the solid was precipitated. At 80 °C, the solid was dissolved to obtain a clear solution, cooled to 60 to 65 °C, and the seed crystal (60 mg, 100 % ee) was added thereto, and the mixture was stirred for 1 hour. The mixture was cooled to 25 °C and stirred for 16 hours, and the solid was filtered to obtain the salt of chiral pure **4-5-K.** The salt was dissolved with 2 N sodium hydroxide solution, extracted with methyl *tert*-butyl ether, dried, and the organic phase was distilled under reduced pressure to give **4-5-K.** EE value was 99.5 %; retention time was 3.976 minutes, SFC analysis method: column type: Chiralpak IC-3 150¡Á4.6 mm I.D., 3 $\mu$m, mobile phase: A: $CO_2$ B: ethanol (0.05 % diethylamine, v/v), elution gradient: B from 5 % to 40 % in 5 minutes and held B at 40 % for 2.5 minutes, and then held B at 5 % for 2.5 minutes, flow rate: 2.5 mL/min, column temperature:

35 °C, column pressure: 1500 psi.

**Synthesis of 4-5:**

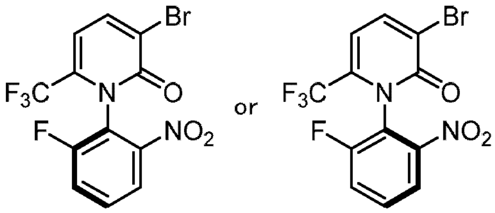

or

**4-5**

Pyridine (18.62 g, 4.07 *eq)* was added to a solution of **4-5-K** (20 g) in tetrahydrofuran (160 mL) at 80 °C, and then a solution of liquid bromine (18.6 g, 2.01 *eq)* in tetrahydrofuran (40 mL) was added dropwise thereto. The mixture was stirred at 80 °C for 16 hours. The generation of the product was detected by LC-MS. The organic phase was distilled under reduced pressure to give a residue. The residue was diluted with ethyl acetate (120 mL) and filtered. The filtrate was washed with 1 M HCl solution (30 mL) and water (30 mL). The organic phase was washed with anhydrous sodium sulfate, concentrated to give a crude product of **4-5.** The crude product was slurried with methyl *tert*-butyl ether (6 mL) and petroleum ether (12 mL) to obtain an intermediate **4-5.** LCMS m/z: 380.8 $[M+1]^+$. EE value was 99.5 %; retention time was 1.206 minutes, SFC analysis method: column type: Chiralcel OJ-3, 100×4.6 mm I.D., 3 μm. Mobile phase: A: $CO_2$ B: isopropanol (0.05 % isopropylamine, v/v). elution gradient: 0.0 minute A 95 % B 5 %, 0.5 minutes; A 95 % B 5 %, 2.0 minutes; A 60 % B 40 %, 3.0 minutes; A 60 % B 40 %, 3.6 minutes; A 95 % B 5 %, 4.0 minutes, A 95 % B 5 %, flow rate: 3.4 mL/min, column temperature: 35 °C, column pressure: 1800 psi.

**Synthesis of 4-3:**

Under nitrogen protection, 1,1-bis(diphenylphosphino)ferrocene palladium dichloride (892.36 mg, 1.22 mmol) and potassium carbonate (3.37 g, 24.39 mmol) were added once to a mixed solution of **compound 4-1** (2.00 g, 12.20 mmol) and **compound 4-2** (8.20 g, 48.78 mmol) in dioxane (20 mL) and water (5 mL), and the mixture was stirred at 85 °C for 12 hours. The reaction solution was diluted with water (50 mL), extracted with ethyl acetate (50 mL×2), and then washed with saturated brine (50 mL). The organic phase was dried over anhydrous sodium sulfate, filtered, concentrated, and separated by silica gel column chromatography (petroleum ether: ethyl acetate of 1: 0 to 3: 1 (v/v)) to obtain **compound 4-3.**

**Synthesis of 4-4:**

**Compound 4-3** (1.90 g, 10.84 mmol) was dissolved in methanol (20 mL) solution. Wet palladium on carbon (190 mg, 10 % purity) was added to the solution, and hydrogen (15 Psi) was introduced thereto, and the mixture was stirred at 25 °C for 12 hours. The reaction solution was filtered with diatomite and concentrated to obtain **compound 4-4.** LCMS (ESI) m/z: 180.2 (M+1).

**Synthesis of 4-6:**

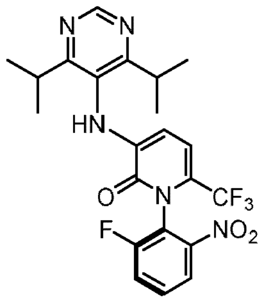

or

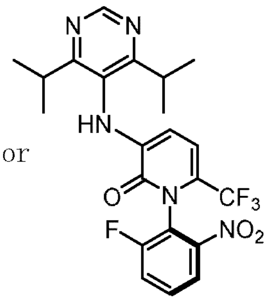

**4-6**

Under nitrogen protection, tris(dibenzylideneacetone)dipalladium (664.08 mg, 0.725 mmol), 4,5-bis(diphenylphosphino)-9,9-dimethylxanthene (839.23 mg, 1.45 mmol) and cesium carbonate (4.73 g, 14.50 mmol) were added to a mixed solvent of **compound 4-4** (1.30 g, 7.25 mmol) and **compound 4-5** (2.76 g, 7.25 mmol) in dioxane (30 mL). The reaction solution was stirred at 100 °C for 12 hours. The reaction solution was diluted with water (100 mL), extracted with ethyl acetate (50 mL×2), and then washed with saturated brine (100 mL). The organic phase was dried over anhydrous sodium sulfate, filtered, concentrated, and separated by silica gel column chromatography (petroleum ether: ethyl acetate of 10: 1 to 1: 1 (v/v)) to obtain **compound 4-6.** LCMS (ESI) m/z: 480.1 (M+1).

**Synthesis of 4-7:**

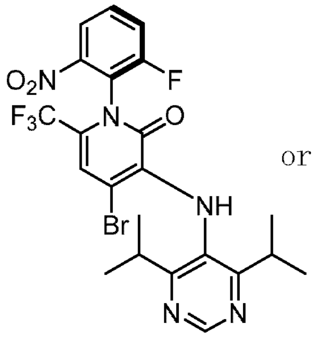

or

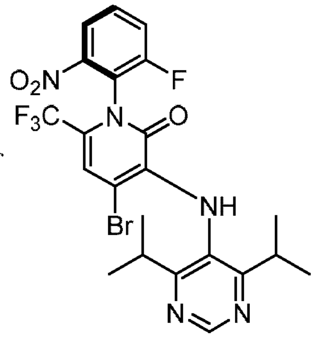

**4-7**

*N*-Bromosuccinimide (928.11 mg, 5.21 mmol) was added to a solution of **compound 4-6** (2.5 g, 5.21 mmol) in acetic acid (30 mL). The reaction solution was stirred at 25 °C for 0.5 hours. After the reaction was completed, the reaction was quenched with saturated sodium sulfite solution (10 mL), and the pH of the solution was adjusted to 8 with saturated sodium bicarbonate solution, then extracted with ethyl acetate (50 mL), and then washed with saturated brine (50 mL). The organic phase was dried over anhydrous sodium sulfate, filtered and concentrated to obtain **compound 4-7.** LCMS (ESI) m/z: 558.0 (M+1).

**Synthesis of 4-9:**

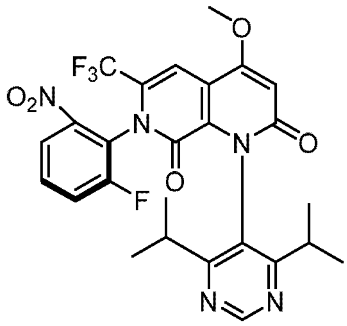

or

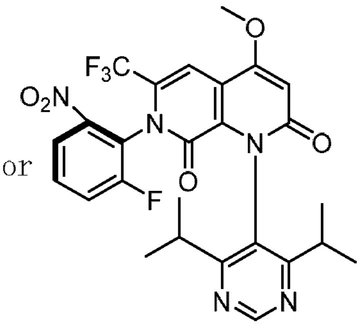

**4-9**

Chloro[(tri-*tert*-butylphosphine)-2-(2-aminobiphenyl)]palladium(II) (384.75 mg, 0.68 mmol) and *N,N*-dicyclohexylmethylamine (731.24 mg, 3.74 mmol) were added to a mixed solvent of **compound 4-7** (1.90 g, 3.40 mmol) and **compound 1-5** (434.66 mg, 3.74 mmol) in toluene (20 mL) under nitrogen protection. The reaction solution was stirred at 110 °C for 16 hours. The reaction solution was concentrated, then diluted with water (100 mL), extracted with ethyl acetate (100 mL×2), and then washed with saturated brine (100 mL). The organic phase was dried over anhydrous sodium sulfate, filtered, concentrated, and separated by silica gel column chromatography (petroleum ether: ethyl acetate of 3: 1 to 1: 1 (v/v)) to obtain **compound 4-9.** LCMS (ESI) m/z: 562.1 (M+1).

**Synthesis of 4-10:**

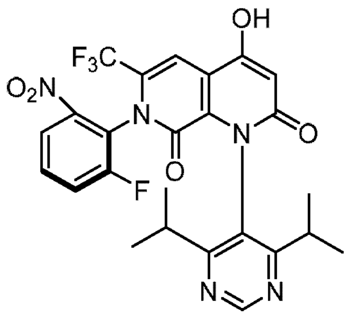

or

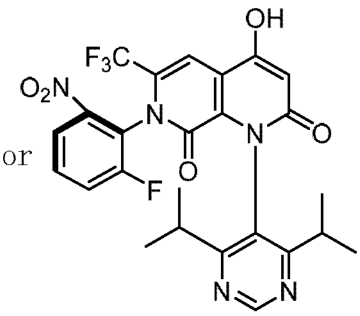

**4-10**

**Compound 4-9** (1.30 g, 2.32 mmol) was added to aqueous hydrochloric acid solution (12 mol/L, 30 mL). The reaction solution was stirred at 115 °C for 36 hours. After the reaction was completed, the reaction solution was concentrated, then diluted with water (100 mL), extracted with ethyl acetate (150 mL×2), and then washed with saturated brine (100 mL). The organic phase was dried over anhydrous sodium sulfate, filtered, concentrated, and separated by silica gel column chromatography (petroleum ether: ethyl acetate of 2: 1 to 0: 1 (v/v)) to obtain **compound 4-10.** LCMS (ESI) m/z: 548.0 (M+1).

**Synthesis of 4-11:**

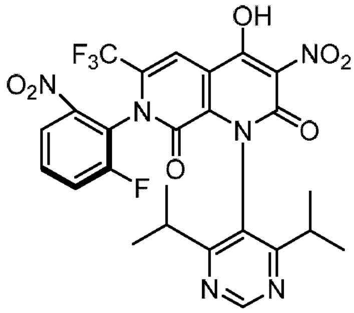

or

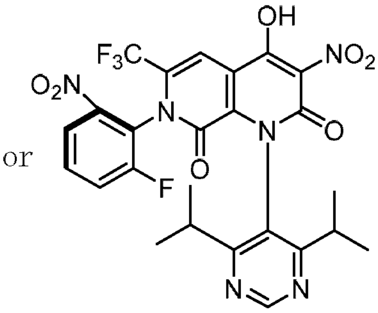

**4-11**

**Compound 4-10** (0.7 g, 1.28 mmol) was added to a mixed solution of acetic acid (10 mL) and fuming nitric acid (1.26 g, 20.00 mmol) at 0 °C, then sodium nitrite (441.10 mg, 6.39 mmol) was added thereto, and the mixture was stirred at 25 °C for 0.5 hours. The reaction solution was diluted with ice water (50 mL), extracted with ethyl acetate (50 mL× 2), and then washed with saturated brine (50 mL). The pH of the obtained organic phase was adjusted to 6 with saturated aqueous sodium bicarbonate solution, and the organic phase was dried over anhydrous sodium sulfate, filtered, and concentrated to obtain **compound 4-11.** LCMS (ESI) m/z: 593.0 (M+1).

**Synthesis of 4-12:**

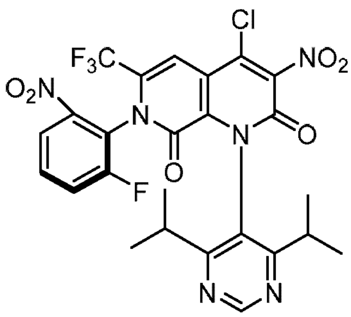

or

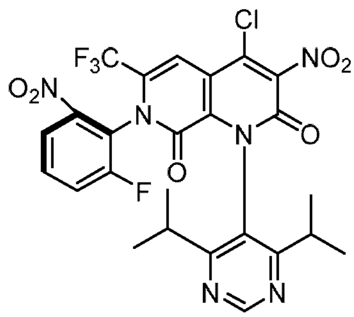

**4-12**

Phosphorous oxychloride (985.83 mg, 5.95 mmol) and *N*,*N*-dimethylformamide (863.62 μg, 11.82 μmol) were added to a solution of **compound 4-11** (0.70 g, 1.18 mmol) in acetonitrile (10 mL) at room temperature, and the mixture was stirred at 45 °C for 1 hour. The reaction solution was diluted with water (50 mL), extracted with ethyl acetate (50 mL), and then washed with saturated brine (50 mL). The organic phase was dried over anhydrous sodium sulfate, filtered, and concentrated to obtain **compound 4-12.** LCMS (ESI) m/z: 611.0 (M+1).

**Synthesis of 4-14:**

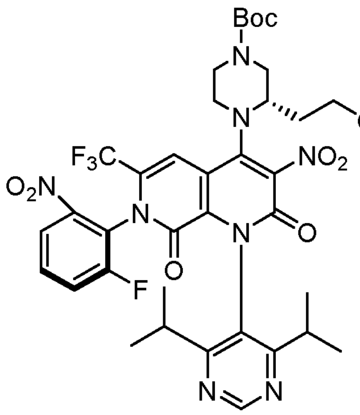

or

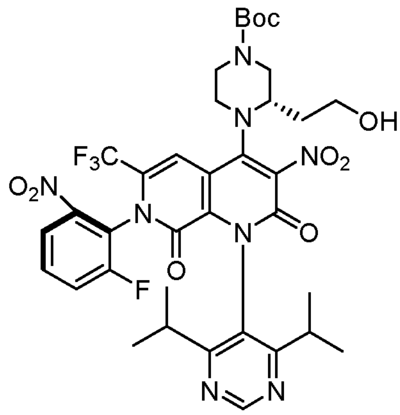

**4-14**

A 4 A molecular sieve (1 g) was added to a solution of **compound 4-12** (0.60 g, 0.982 mmol) and **compound 4-13** (452.39 mg, 1.96 mmol) in acetonitrile (10 mL), and the reaction solution was stirred at 45 °C for 12 hours. The reaction solution was filtered with diatomite and concentrated, then diluted with water (20 mL), extracted with ethyl acetate (20 mL×2),and then washed with saturated brine (20 mL). The organic phase was dried over anhydrous sodium sulfate, filtered, concentrated, and separated by silica gel column chromatography (petroleum ether: ethyl acetate of 2:1 to 1:1 (v/v)) to obtain **compound 4-14.** LCMS (ESI) m/z: 805.1 (M+1).

**Synthesis of 4-15:**

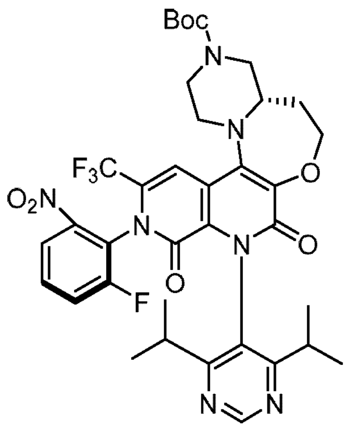

or

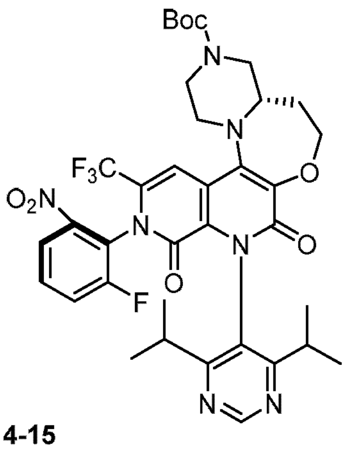

**4-15**

1,8-Diazabicyclo[5.4.0]undec-7-ene (151.34 mg, 0.994 mmol) was added to a solution of **compound 4-14** (0.4 g, 0.497 mmol) in dry DMF (8 mL). The reaction solution was stirred at 130 °C for 30 minutes. The reaction solution was diluted with ethyl acetate (50 mL), washed with water (30 mL×3) and saturated brine (20×2 mL), and the organic phase was dried over anhydrous sodium sulfate to obtain **compound 4-15.** LCMS (ESI) m/z: 758.2 (M+1).

**Synthesis of 4-16:**

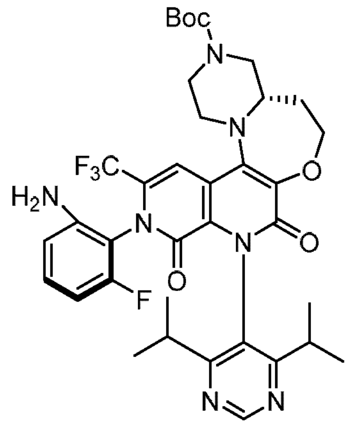

or

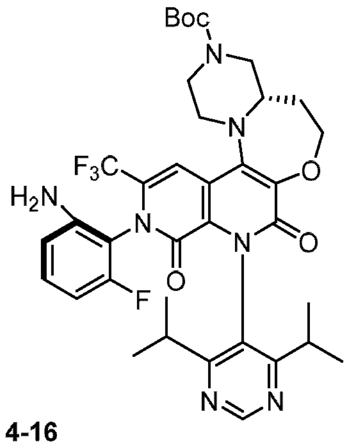

**4-16**

**Compound 4-15** (0.35 g, 0.461 mmol) was added to a mixed solution of ethanol (5 mL) and water (3 mL), and then ammonium chloride (247.08 mg, 4.62 mmol) and iron powder (257.95 mg, 4.62 mmol) were added to the above mixed solution and stirred at 80 °C for 2 hours. The reaction solution was filtered, and the filtrate was diluted with water (20 mL), and extracted with ethyl acetate (30 mL×2). The organic phase was washed with saturated brine (20 mL), then dried over sodium sulfate, filtered, and the filtrate was concentrated to obtain **compound 4-16.** LCMS (ESI) m/z: 728.2 (M+1).

**Synthesis of 4-17:**

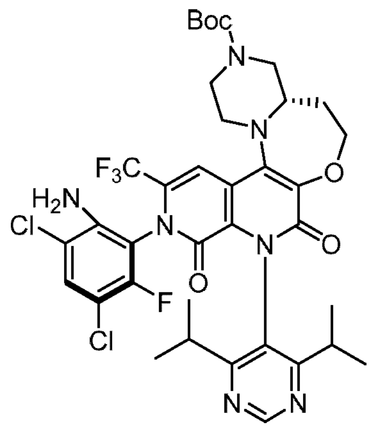

or

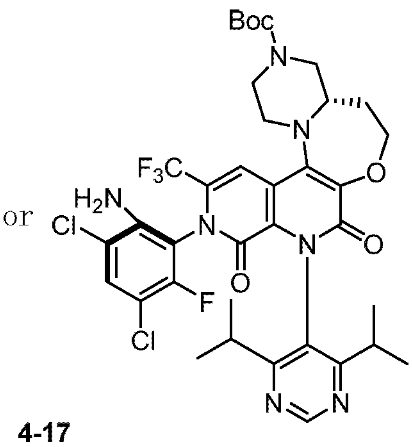

**4-17**

**Compound 4-16** (0.18 g, 0.247 mmol) was dissolved in DMF (10 mL) and THF (5 mL), and then cooled to 0 °C. DCDMH (82.84 mg, 0.42 mmol) was added to the above mixed solution and the mixture was stirred for 30 minutes. The reaction solution was diluted with water (20 mL), extracted with ethyl acetate (20 mL×2), and the organic phase was washed with saturated brine (20 mL), dried over sodium sulfate, filtered, and the filtrate was concentrated to obtain the crude product of **compound 4-17.** LCMS (ESI) m/z: 796.1 (M+1).

**Synthesis of 4-18:**

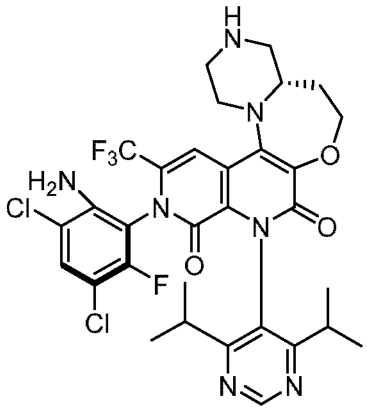

or

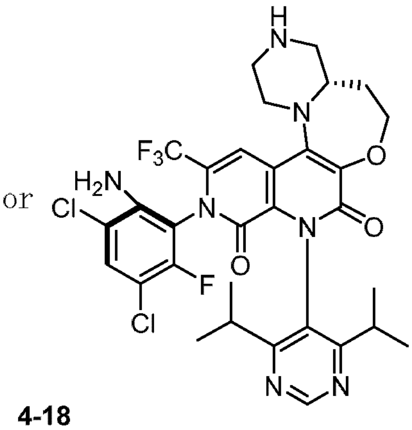

**4-18**

**Compound 4-17** (200 mg, 0.25 mmol) was dissolved in dichloromethane (3.0 mL), and then trifluoroacetic acid (1 mL) was added thereto at 25 °C and stirred for 20 minutes. The reaction solution was concentrated to obtain the crude product of **compound 4-18.**

**Synthesis of compound 4:**

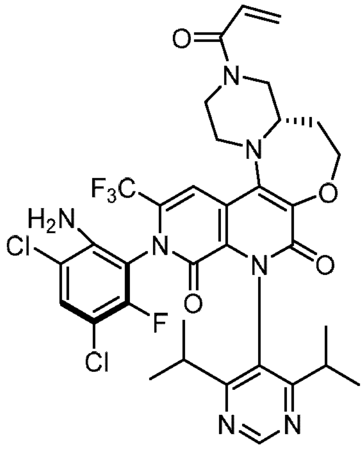

or

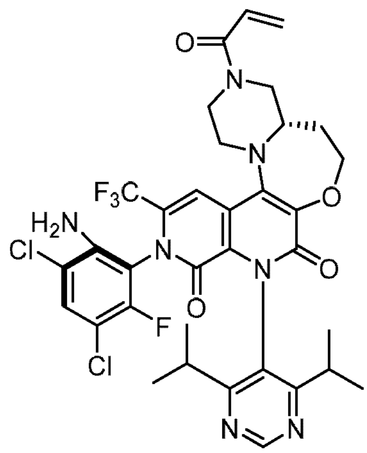

**4**

**Compound 4-18** (200 mg, 0.287 mmol) was dissolved in a mixed solution of THF (4 mL) and water (2 mL), and the pH of the above solution was adjusted to 8 with potassium carbonate, and then acryloyl chloride (38.98 mg, 0.43 mmol) was added dropwise to the reaction solution and the mixture was stirred for 5 minutes. The reaction solution was diluted with water (20 mL), extracted with ethyl acetate (20 mL×2), and the organic phase was washed with saturated brine (20 mL), dried over sodium sulfate, and filtered. The filtrate was concentrated and then separated by preparative HPLC (chromatographic column: Phenomenex luna C18 150*25 mm*10 μm; mobile phase: [water (0.225 % formic acid)-acetonitrile]; acetonitrile %: 49 %-69 %, 10 minutes) to obtain **compound 4.** LCMS (ESI) m/z: 750.1 (M+1). **$^1$HNMR** (400 MHz, DMSO-$d_6$) δ = 8.98 (s, 1H), 7.63 (d, J = 7.6 Hz, 1H), 7.2 (s, 1H), 6.99 - 6.74 (m, 1H), 6.25 - 6.07 (m, 3H), 5.81 - 5.72 (m, 1H), 4.71 - 4.35 (m, 2H), 4.18 (dd, J = 4.8, 8.4 Hz, 2H), 4.02 - 3.70 (m, 2H), 3.66 - 3.43 (m, 3H), 2.92 - 2.74 (m, 1H), 2.14 (s, 2H), 1.13 - 0.83 (m, 13H).

**Embodiment 5**

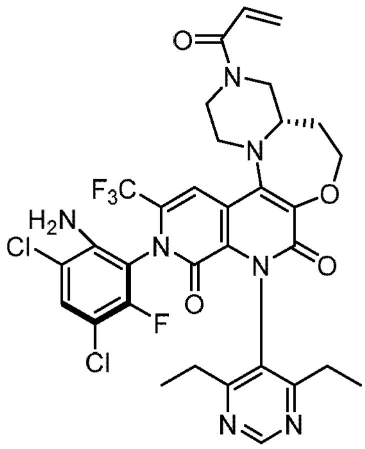

5A or 5B

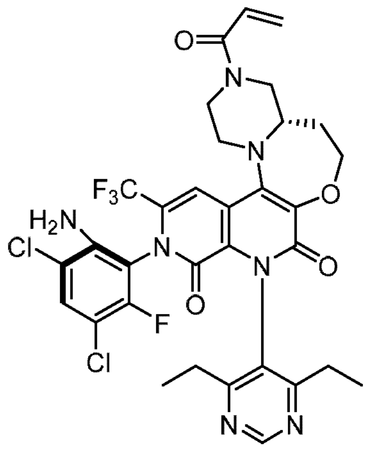

5B or 5A

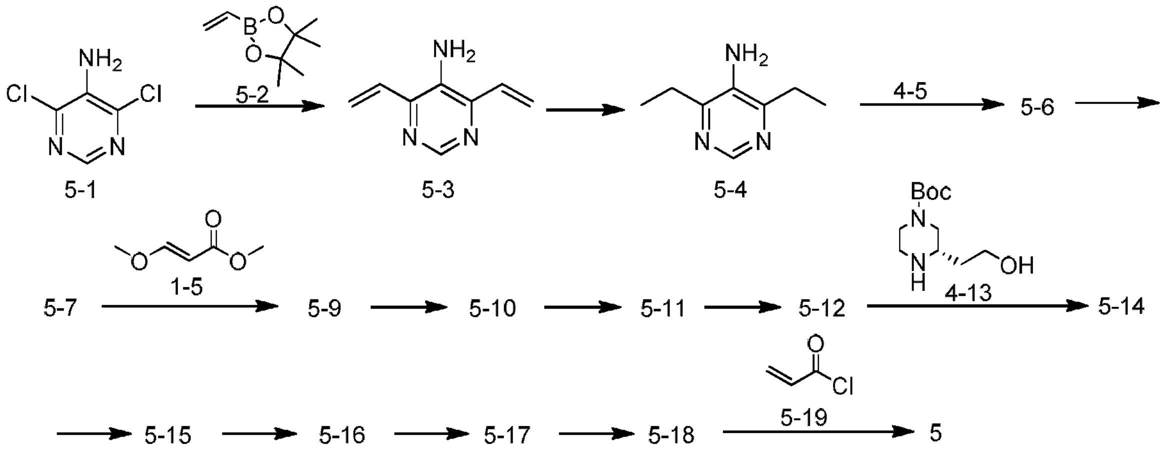


**Synthesis of 5-3:** Under nitrogen protection, 1,1-bis(diphenylphosphino)ferrocene palladium dichloride (2.5 g, 3.41 mmol) and potassium carbonate (35.40 g, 256.11 mmol) were added once to a mixed solution of **compound 5-1** (14 g, 85.37 mmol) and **compound 5-2** (52.59 g, 341.48 mmol) in dioxane (150 mL) and water (30 mL), and the mixture was stirred at 85 °C for 12 hours. The reaction solution was concentrated, then diluted with water (500 mL), extracted with ethyl acetate (500 mL×2), and then washed with saturated brine (200 mL). The organic phase was dried over anhydrous sodium sulfate, filtered, concentrated, and separated by silica gel column chromatography (petroleum ether: ethyl acetate of 1: 0 to 1: 1 (v/v)) to obtain **compound 5-3.**

**Synthesis of 5-4: Compound 5-3** (5.7 g, 38.73 mmol) was dissolved in methanol (50 mL) solution. Wet palladium on carbon (500 mg, 10 % purity) was added to the solution, and hydrogen (15 Psi) was introduced thereto, and the mixture was stirred at 25 °C for 12 hours. The reaction solution was filtered with diatomite and concentrated to obtain **compound 5-4.** LCMS (ESI) m/z: 152.2 (M+1).

**Synthesis of 5-6:**

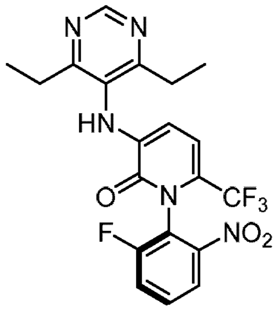

or

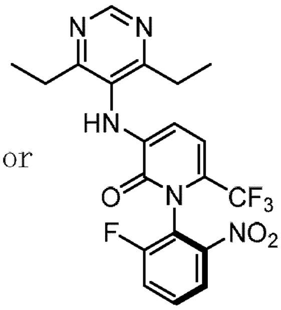

**5-6**

Under nitrogen protection, tris(dibenzylideneacetone)dipalladium (2.24 g, 2.45 mmol), 4,5-bis(diphenylphosphino)-9,9-dimethylxanthene (2.83 g, 4.89 mmol) and cesium carbonate (15.95 g, 48.94 mmol) were added to a mixed solvent of **compound 5-4** (3.7 g, 24.47 mmol) and **compound 4-5** (10.26 g, 26.92 mmol) in dioxane (30 mL). The reaction solution was stirred at 100 °C for 12 hours. The reaction solution was diluted with water (100 mL), extracted with ethyl acetate (50 mL×2), and then washed with saturated brine (100 mL). The organic phase was dried over anhydrous sodium sulfate, filtered, concentrated, and then separated by silica gel column chromatography (petroleum ether: ethyl acetate of 5: 1 to 1: 1 (v/v)) to obtain **compound 5-6.** LCMS (ESI) m/z: 452.1 (M+1).

**Synthesis of 5-7:**

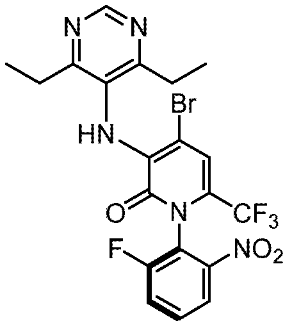

or

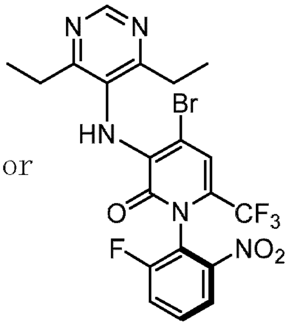

**5-7**

N-Bromosuccinimide (3.19 g, 17.95 mmol) was added to a solution of **compound 5-6** (5.4 g, 11.96 mmol) in acetic acid (50 mL). The reaction solution was stirred at 25 °C for 0.5 hours. After the reaction was completed, the reaction was quenched with saturated sodium sulfite solution (50 mL), and the pH of the solution was adjusted to 8 with sodium hydroxide, then extracted with ethyl acetate (200 mL), and then washed with saturated brine (100 mL). The organic phase was dried over anhydrous sodium sulfate, filtered, concentrated and then separated by silica gel column chromatography (petroleum ether: ethyl acetate of 4:1 to 2:1 (v/v)) to obtain **compound 5-7.** LCMS (ESI) m/z: 529.9 (M+1).

**Synthesis of 5-9:**

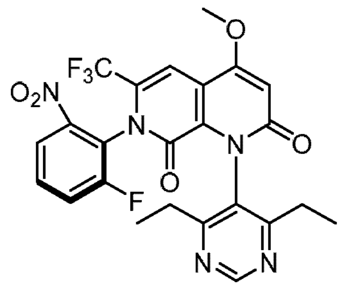

or

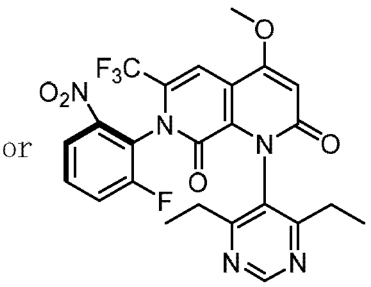

**5-9**

Chloro[(tri-*tert*-butylphosphine)-2-(2-aminobiphenyl)]palladium(II) (289.89 mg, 0.565 mmol) and *N*,*N*-dicyclohexylmethylamine (607.84 mg, 3.11 mmol) were added to a mixed solvent of **compound 5-7** (1.5 g, 2.83 mmol) and **compound 1-5** (492.69 mg, 4.24 mmol) in toluene (15 mL) under nitrogen protection. The reaction solution was stirred at 110 °C for 16 hours. The reaction solution was concentrated, then diluted with water (100 mL), extracted with ethyl acetate (100 mL×2), and then washed with saturated brine (100 mL). The organic phase was dried over anhydrous sodium sulfate, filtered, concentrated, and separated by silica gel column chromatography (petroleum ether: ethyl acetate of 3: 1 to 1: 1 (v/v)) to obtain **compound** 5-9. LCMS (ESI) m/z: 534.1 (M+1).

**Synthesis of 5-10:**

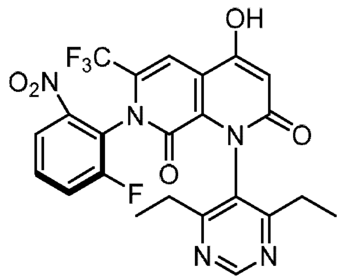

or

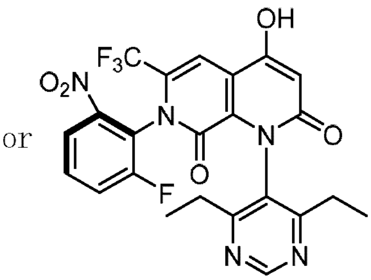

**5-10**

**Compound** 5-9 (0.5 g, 0.937 mmol) was added to aqueous hydrochloric acid solution (12 mol/L, 20 mL). The reaction solution was stirred at 115 °C for 36 hours. After the reaction was completed, the reaction solution was concentrated, then diluted with water (50 mL), extracted with ethyl acetate (50 mL×2), and then washed with saturated brine (50 mL). The organic phase was dried over anhydrous sodium sulfate, filtered, and concentrated to obtain **compound 5-10.** LCMS (ESI) m/z: 520.0 (M+1).

**Synthesis of 5-11:**

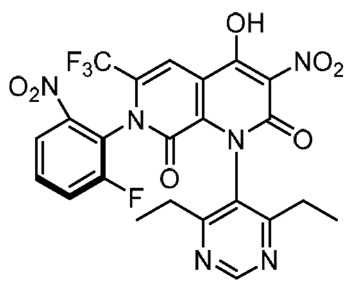

or

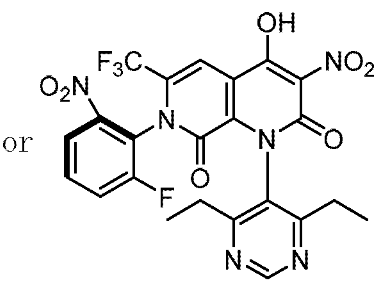

**5-11**

**Compound 5-10** (0.40 g, 0.77 mmol) was added to a mixed solution of acetic acid (5 mL) and fuming nitric acid (0.96 g, 15.23 mmol) at 0 °C, then sodium nitrite (265.67 mg, 3.85 mmol) was added thereto, and the mixture was stirred at 25 °C for 0.5 hours. The reaction solution was diluted with ice water (50 mL), extracted with ethyl acetate (20 mL× 2), and then washed with saturated brine (20 mL× 2). The pH of the obtained organic phase was adjusted to 6 with saturated aqueous sodium bicarbonate solution, and the organic phase was dried over anhydrous sodium sulfate, filtered, and concentrated to obtain **compound 5-11.** LCMS (ESI) m/z: 565.0 (M+1).

**Synthesis of 5-12:**

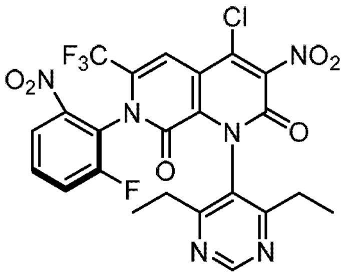
or
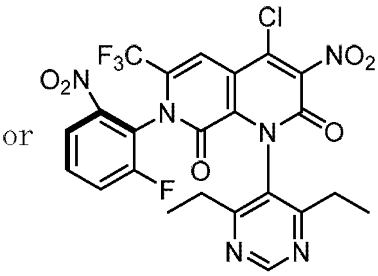

**5-12**

Phosphorous oxychloride (407.51 mg, 2.66 mmol) and *N,N*-dimethylformamide (388.52 μg, 5.32 μmol) were added to a solution of **compound 5-11** (0.30 g, 0.53 mmol) in acetonitrile (3 mL) at room temperature, and the mixture was stirred at 45°C for 1 hour. The reaction solution was diluted with water (10 mL), extracted with ethyl acetate (10 mL), and then washed with saturated brine (10 mL). The organic phase was dried over anhydrous sodium sulfate, filtered, and concentrated to obtain **compound 5-12.** LCMS (ESI) m/z: 583.0 (M+1).

**Synthesis of 5-14:**

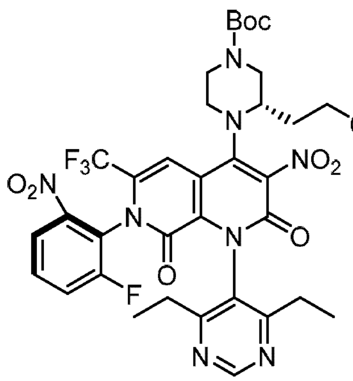
or
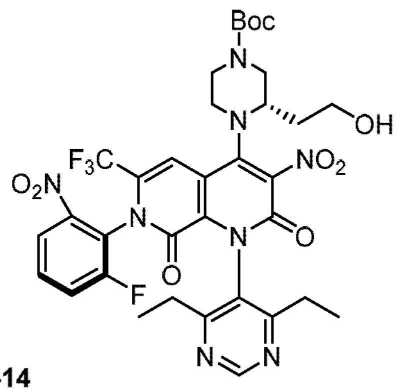

**5-14**

A 4A molecular sieve (0.5 g) was added to a solution of **compound 5-12** (0.28 g, 0.48 mmol) and **compound 4-13** (221.28 mg, 0.96 mmol) in acetonitrile (3 mL), and the reaction solution was stirred at 45 °C for 12 hours. The reaction solution was filtered with diatomite and concentrated, then diluted with water (20 mL), extracted with ethyl acetate (20 mL×2), and then washed with saturated brine (20 mL). The organic phase was dried over anhydrous sodium sulfate, filtered, concentrated, and separated by silica gel column chromatography (petroleum ether: ethyl acetate of 2: 1 to 0: 1 (v/v)) to obtain **compound 5-14.** LCMS (ESI) m/z: 777.1 (M+1).

**Synthesis of 5-15:**

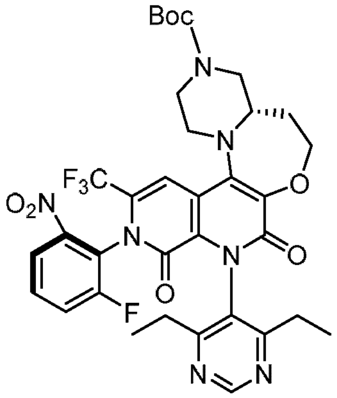
or
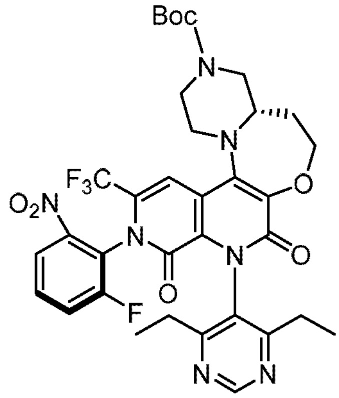

**5-15**

1,8-Diazabicyclo[5.4.0]undec-7-ene (44.10 mg, 0.29 mmol) was added to a solution of **compound 5-14** (0.15 g, 0.19 mmol) in dry DMF (2 mL). The reaction solution was stirred at 130 °C for 30 minutes. The reaction solution was diluted with ethyl acetate (20 mL), washed with water (10 mL×3) and saturated brine (10×2 mL), and the organic phase was dried over anhydrous sodium sulfate to obtain **compound 5-15.** LCMS (ESI) m/z: 730.2 (M+1).

**Synthesis of 5-16:**

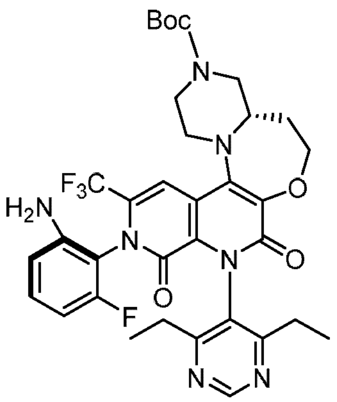
or
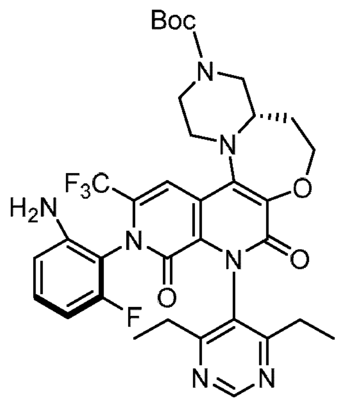

**5-16**

**Compound 5-15** (0.11 g, 0.15 mmol) was added to a mixed solution of ethanol (1 mL) and water (1 mL), and then ammonium chloride (80.64 mg, 1.51 mmol) and iron powder (84.19 mg, 1.51 mmol) were added to the above mixed solution and stirred at 80 °C for 12 hours. The reaction solution was filtered, and the filtrate was diluted with water (10 mL), and extracted with ethyl acetate (10 mL). The organic phase was washed with saturated brine (10 mL), then dried over sodium sulfate, filtered, and the filtrate was concentrated to obtain **compound 5-16.** LCMS (ESI) m/z: 700.2 (M+1).

**Synthesis of 5-17:**

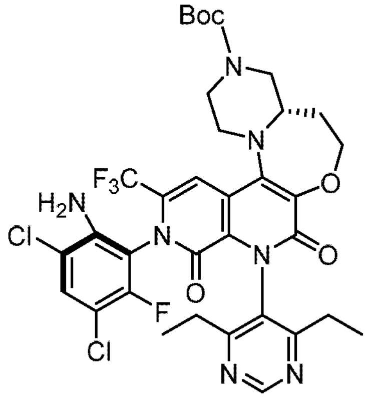

or

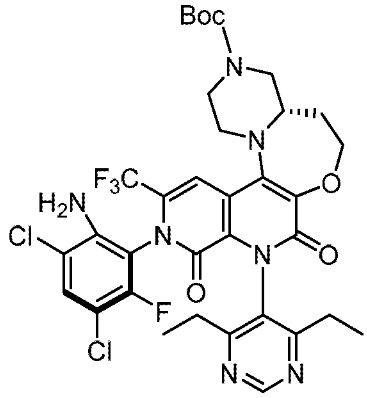

**5-17**

**Compound 5-16** (0.1 g, 0.14 mmol) was dissolved in DMF (2 mL), and then cooled to 0 °C. DCDMH (47.87 mg, 0.24 mmol) was added to the above mixed solution and the mixture was stirred for 30 minutes. The reaction solution was diluted with water (20 mL), extracted with ethyl acetate (20 mL×2), and the organic phase was washed with saturated brine (20 mL), dried over sodium sulfate, filtered, and the filtrate was concentrated to obtain the crude product of **compound 5-17.** LCMS (ESI) m/z: 768.2 (M+1).

**Synthesis of 5-18:**

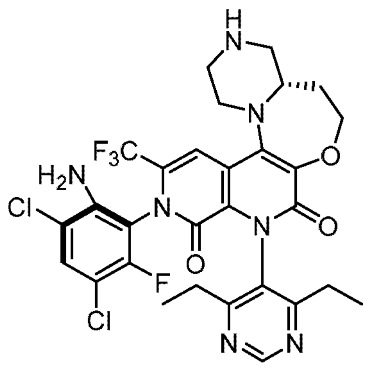

or

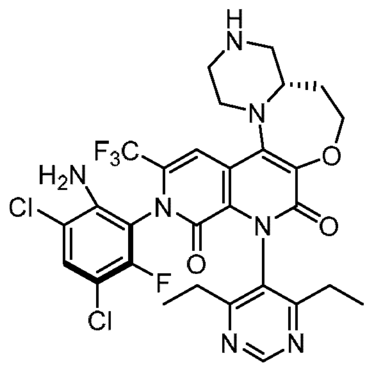

**5-18**

**Compound 5-17** (140 mg, 0.18 mmol) was dissolved in dichloromethane (2 mL), and then trifluoroacetic acid (0.5 mL) was added thereto at 25 °C and stirred for 20 minutes. The reaction solution was concentrated to obtain the crude product of **compound 5-18.**

**Synthesis of compound 5:**

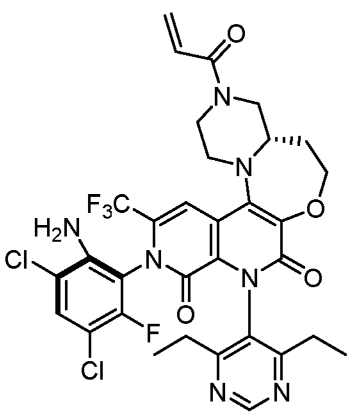

or

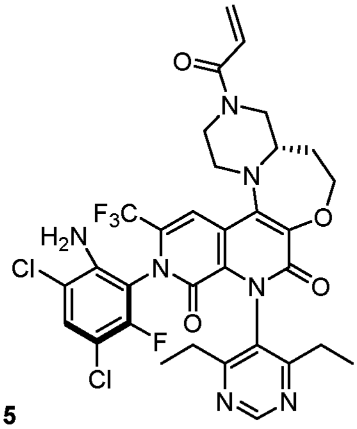

**5**

**Compound 5-18** (120 mg, 0.18 mmol) was dissolved in a mixed solution of THF (2 mL) and water (1 mL), and the pH of the above solution was adjusted to 8 with potassium carbonate, and then acryloyl chloride (24.37 mg, 0.27 mmol) was added dropwise to the reaction solution and the mixture was stirred for 5 minutes. The reaction solution was diluted with water (10 mL), extracted with ethyl acetate (10 mL), and the organic phase was washed with saturated brine (10 mL), dried over sodium sulfate, and filtered. The filtrate was concentrated and then separated by preparative HPLC (chromatographic column: Phenomenex luna C18 150*25 mm*10 μm; mobile phase: [water (0.1 % trifluoroacetic acid)-acetonitrile]; acetonitrile %: 50 %-80 %, 10 minutes) to obtain **compound 5.** LCMS (ESI) m/z: 722.1 (M+1).

**Embodiment 6**

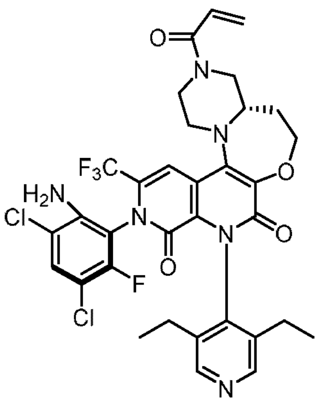

**6A or 6B**

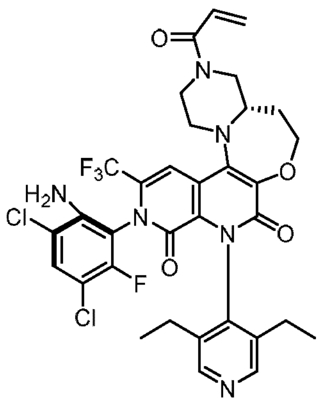

**6B or 6A**

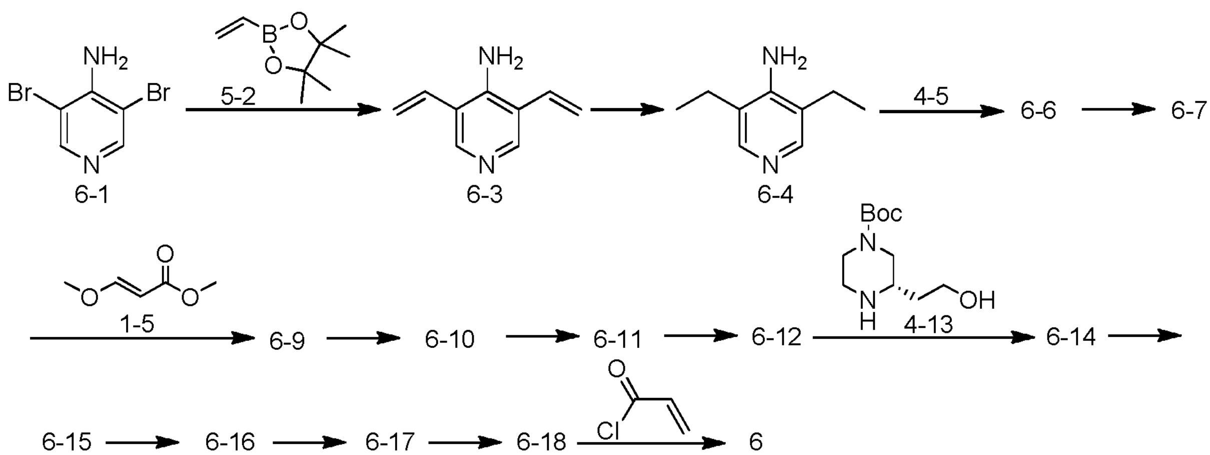


**Synthesis of 6-3:** Under nitrogen protection, Pd(dppf)$Cl_2$ (2.90 g, 3.97 mmol) and potassium carbonate (16.46

g, 119.09 mmol) were added to a solution of **compound 6-1** (10 g, 37.70 mmol) and **compound 5-2** (24.46 g, 158.79 mmol) in dioxane (80 mL) and water (20 mL) at one time, and the mixture was stirred at 100 °C for 12 hours. The reaction solution was concentrated and then separated by silica gel column chromatography (petroleum ether: ethyl acetate of 0: 1 (v/v)) to obtain **compound 6-3.** LCMS (ESI) m/z: 147.2 (M+1).

**Synthesis of 6-4:** Palladium on carbon (0.5 g, 17.10 mmol) was added to a solution of **compound 6-3** (5.2 g, 35.57 mmol) in methanol (20 mL) at 20 °C under nitrogen protection, and the mixture was stirred for 10 minutes under the condition of hydrogen (15 PSI) at 20 °C. The reaction solution was filtered through diatomite and dried to dryness by rotary evaporation to obtain the crude product of **6-4.** LCMS (ESI) m/z: 151.2 (M+1).

**Synthesis of 6-6:**

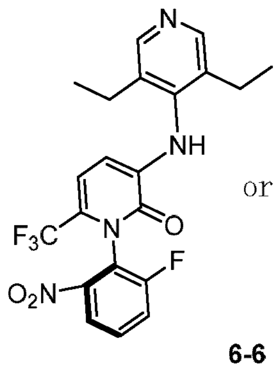

or

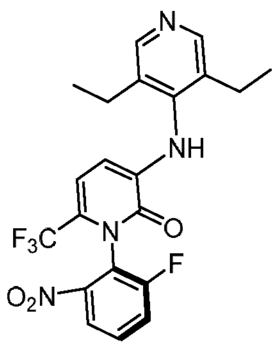

**6-6**

Under nitrogen protection, tris(dibenzylideneacetone)dipalladium (1.52 g, 1.66 mmol), 4,5-bis(diphenylphosphino)-9,9-dimethylxanthene (1.93 g, 3.33 mmol) and cesium carbonate (21.69 g, 66.57 mmol) were added to a mixed solution of **compound 6-4** (5 g, 33.28 mmol) and **compound 4-5** (12.68 g, 32.28 mmol) in dioxane (150 mL). The reaction solution was stirred at 100 °C for 3 hours. The reaction solution was concentrated and then separated by silica gel column chromatography (petroleum ether: ethyl acetate of 1: 1 (v/v)) to obtain **compound 6-6.** LCMS (ESI) m/z: 451.1 (M+1).

**Synthesis of 6-7:**

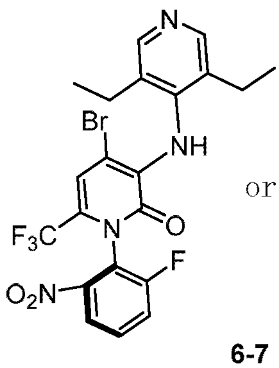

or

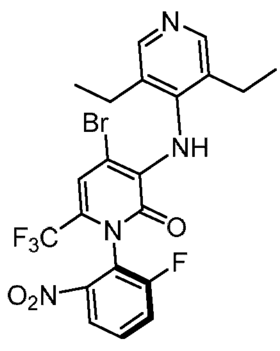

**6-7**

Under nitrogen protection, **compound 6-6** (6 g, 13.323 mmol) was dissolved in ethyl acetate (100 mL), and then NBS (2.37 g, 13.32 mmol) was added thereto in batches. The reaction solution was reacted at 20 °C for 0.5 hours. After the reaction was completed, the mixture was extracted, concentrated, and then separated by silica gel column chromatography (petroleum ether: ethyl acetate of 1: 1 (v/v)) to obtain **compound 6-7.** LCMS (ESI) m/z: 530.9 (M+1). $^1H$ NMR (400 MHz, $CDCl_3$) δ = 8.25 (s, 2H), 8.07 (d, *J* = 8.4 Hz, 1H), 7.66 (dt, *J* = 5.6, 8.4 Hz, 1H), 7.54 (dt, *J* = 1.2, 8.4 Hz, 1H), 6.88 (s, 2H), 2.63 - 2.36 (m, 4H), 1.18 - 1.04 (m, 6H).

**Synthesis of 6-9:**

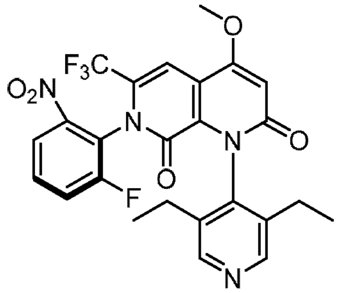

or

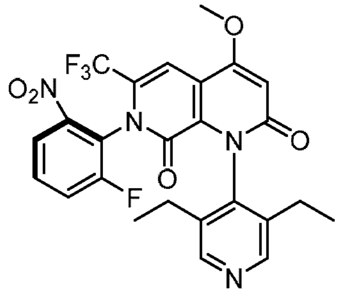

**6-9**

Under nitrogen protection and at 20 °C, **compound 6-7** (5 g, 9.45 mmol) and **compound 1-5** (4.39 g, 37.79 mmol) were added to dioxane (50 mL), and then dicyclohexylmethylamine (2.77 g, 14.17 mmol), lithium chloride (1.6 g, 37.79 mmol) and tri-*tert*-butylphosphine palladium (724.17 mg, 1.42 mmol) were added thereto. The reaction solution was reacted under nitrogen protection at 105 °C for 12 hours. The reaction solution was diluted with ethyl acetate (150 mL), and then washed with saturated brine (50 mL, 3 times). The organic phase was dried over anhydrous sodium sulfate, filtered, concentrated, and then separated by silica gel column chromatography (petroleum ether: ethyl acetate of 0: 1 (v/v)) to obtain **compound 6-9.**

**Synthesis of 6-10:**

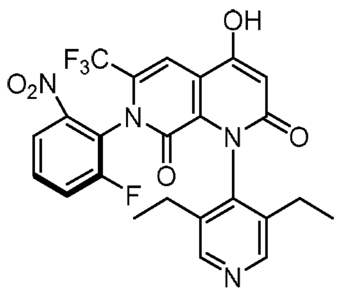

or

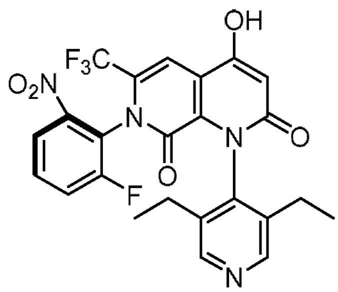

**6-10**

Under nitrogen protection, **compound 6-9** (2 g, 3.76 mmol) was added to aqueous hydrochloric acid solution (12 mol/L, 20 mL). The reaction solution was stirred at 110 °C for 2 hours. After the reaction was completed, the mixture was directly concentrated under reduced pressure, and then extracted with ethyl acetate (25 mL, two times). The organic phase was dried over sodium sulfate, filtered, and concentrated to obtain **compound 6-10.** LCMS (ESI) m/z: 519.0 (M+1).

**Synthesis of 6-11:**

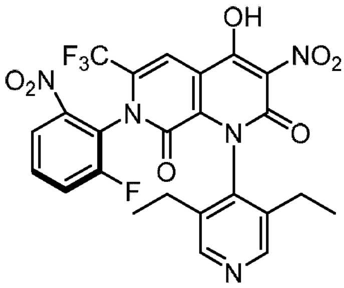

or

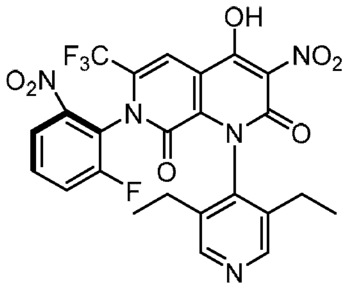

**6-11**

**Compound 6-10** (2 g, 3.86 mmol) was added to a mixed solution of acetic acid (15 mL) and fuming nitric acid (1.22 g, 19.29 mmol) at 0 °C, then sodium nitrite (1.33 g, 19.29 mmol) was added thereto, and the mixture was stirred at 15 °C for 0.5 hours. The reaction solution was diluted with ethyl acetate (50 mL), and then washed with saturated sodium bicarbonate (40 mL). The organic phase was dried over anhydrous sodium sulfate, filtered, and concentrated to obtain **compound 6-11.** LCMS (ESI) m/z: 564.2 (M+1).

**Synthesis of 6-12:**

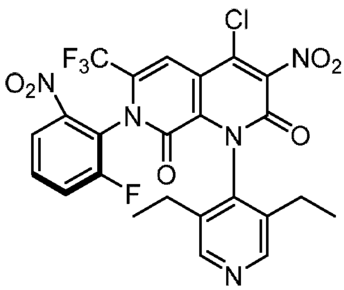

or

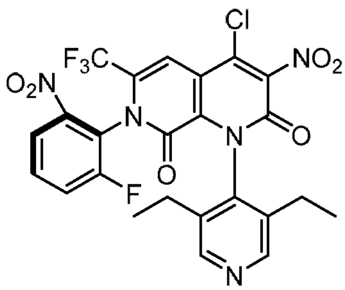

**6-12**

Phosphorus oxychloride (2.52 g, 16.42 mmol) was added to a solution of **compound 6-11** (1.85 g, 3.28 mmol) and DMF (24 mg, 0.33 mmol) in acetonitrile (15 mL) at room temperature under nitrogen protection, and the mixture was stirred at 40 °C for 0.5 hours. After the reaction was completed, the mixture was extracted, concentrated and then separated by silica gel column chromatography (petroleum ether: ethyl acetate of 2: 1 (v/v)) to obtain **compound 6-12.** LCMS (ESI) m/z: 582.0 (M+1).

**Synthesis of 6-14:**

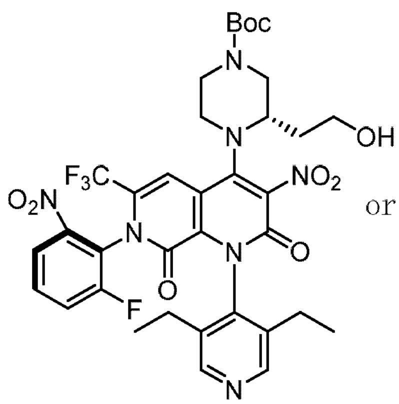

or

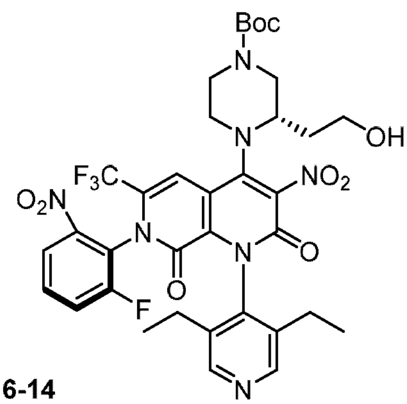

**6-14**

Under nitrogen protection, a 4A molecular sieve (2 g) was added to a solution of **compound 6-12** (0.5 g, 0.859 mmol) and **compound 4-13** (435.39 mg, 1.89 mmol) in acetonitrile (15 mL), and the reaction solution was stirred at 45 °C for 12 hours. The reaction solution was filtered through diatomite, extracted with ethyl acetate (20 mL × 2) and water (20 mL), concentrated and then separated by silica gel column chromatography (petroleum ether: dichloromethane: ethyl acetate of 6: 7: 8 (v/v)) to obtain **compound 6-14.** LCMS (ESI) m/z: 776.5 (M+1).

**Synthesis of 6-15:**

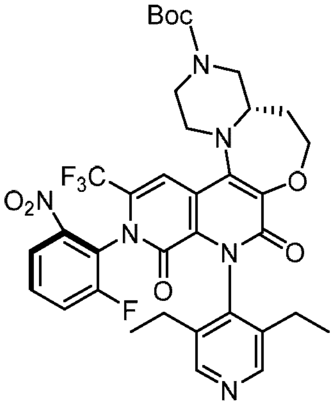

or

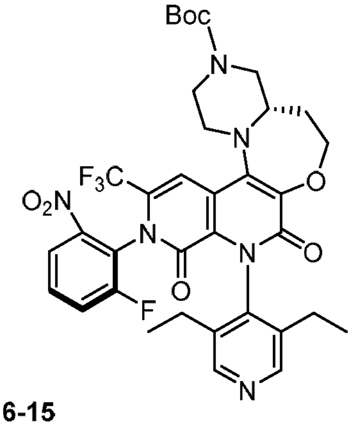

**6-15**

Under nitrogen protection, 1,8-diazabicyclo[5.4.0]undec-7-ene (88.32 mg, 580.12 μmol) was added to a solution of **compound 6-14** (300 mg, 386.75 μmol) in dry DMF (3 mL). The reaction solution was stirred at 130 °C for 0.5 hours. LCMS showed that the reaction was completed. The reaction solution was diluted with ethyl acetate (20 mL), washed with water (20 mL), concentrated, and the residue was separated by silica gel column chromatography (petroleum ether: ethyl acetate of 0: 1 (v/v)) to obtain **compound 6-15.** LCMS (ESI) m/z: 729.1 (M+1).

**Synthesis of 6-16:**

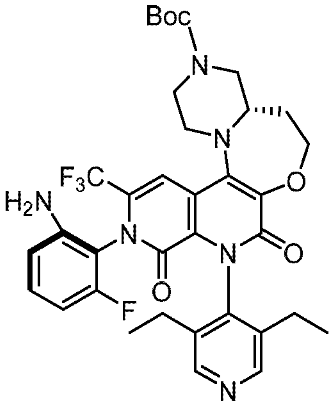

or

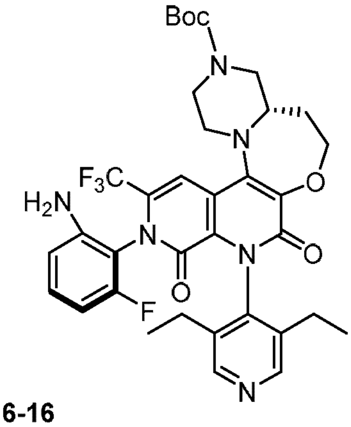

**6-16**

**Compound 6-15** (0.12 g, 164.68 μmol) was added to a mixed solution of ethanol (4 mL) and water (2 mL), and then ammonium chloride (44.04 mg, 823.40 μmol) and iron powder (45.99 mg, 823.40 μmol) were added to the above mixed solution, and the mixture was stirred at 80 °C for 0.5 hours. The reaction solution was filtered and dried to dryness by rotary evaporation, and the residue was separated by silica gel column chromatography (petroleum ether: ethyl acetate of 0: 1 (v/v)) to obtain **compound 6-16.** LCMS (ESI) m/z: 699.2 (M+1).

**Synthesis of 6-17:**

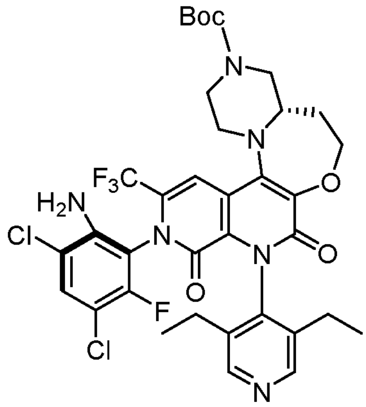

or

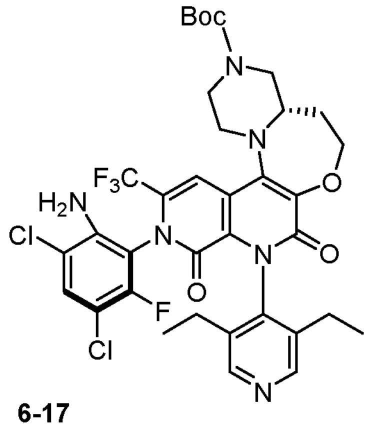

**6-17**

**Compound 6-16** (50 mg, 71.56 μmol) was dissolved in DMF (2 mL) and ethyl acetate (2 mL), and then cooled to 0 °C. DCDMH (23.97 mg, 121.65 μmol) was added to the above mixed solution and the mixture was stirred for 10 minutes. The reaction solution was diluted with ethyl acetate (20 mL), washed with saturated sodium bisulfite (10 mL×2) and saturated sodium bicarbonate (10 mL), and concentrated to obtain the crude product of **compound 6-17.** LCMS (ESI) m/z: 767.1 (M+1).

**Synthesis of 6-18:**

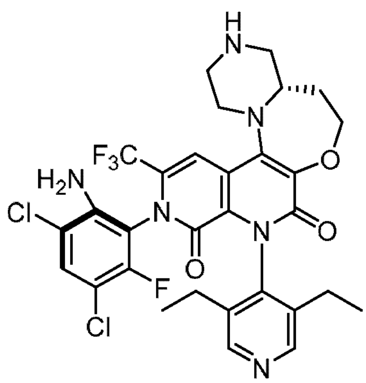

or

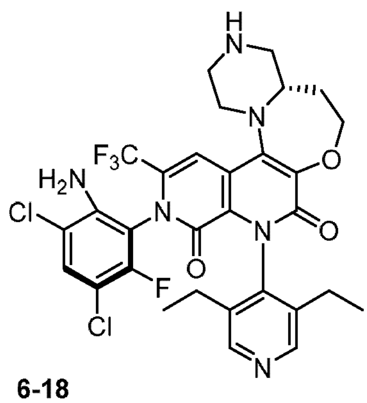

**6-18**

**Compound 6-17** (50 mg, 65.14 mmol) was dissolved in dichloromethane (3.0 mL), cooled to 0 to 5 °C, and trifluoroacetic acid (1.54 g, 13.51 mmol) was added thereto and the mixture was stirred at 15 °C for 15 minutes. The reaction solution was directly concentrated under reduced pressure to obtain **compound 6-18.** LCMS (ESI) m/z: 667.4 (M+1).

**Synthesis of compound 6:**

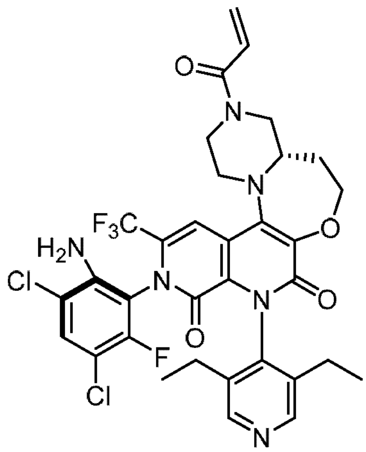

or

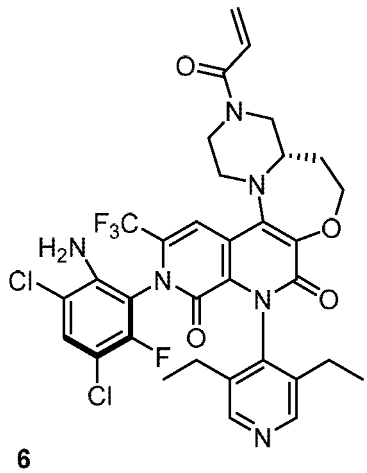

**6**

Under nitrogen protection, potassium carbonate (20.71 mg, 149.82 μmol) and acryloyl chloride (8.14 mg, 89.89 μmol) were added to a solution of **compound 6-18** (50 mg, 65.14 μmol) in tetrahydrofuran (5 mL) and water (2 mL), and the mixture was stirred at 25 °C for 30 minutes. The reaction solution was extracted with water (10 mL) and ethyl acetate (50 mL×2). The combined organic phases were dried over sodium sulfate, filtered and concentrated to obtain the crude product of compound 6. The crude product was separated by prep-HPLC (column information: Phenomenex Gemini-NX C18 75*30 mm*3 μm; mobile phase: [water (0.225 % formic acid)-acetonitrile]; acetonitrile %: 32 %-42 %, 7 minutes) to obtain compound **6.** LCMS (ESI) m/z: 721.1(M+1).

$^1H$ NMR (400 MHz, $CDCl_3$) δ = 8.45 (br d, *J* = 8.5 Hz, 2H), 7.42 (d, *J* = 7.0 Hz, 1H), 7.27 - 7.16 (m, 1H), 6.73 - 6.35 (m, 2H), 5.89 - 5.78 (m, 1H), 5.07 - 4.59 (m, 1H), 4.30 - 3.83 (m, 4H), 3.75 - 2.93 (m, 3H), 2.51 - 2.17 (m, 7H), 1.22 - 0.88 (m, 8H).

**Experimental embodiment 1: Anti-proliferation experiment of H358 cells**

**Experimental materials:**

RPMI-1640 culture medium, penicillin/streptomycin antibiotics purchased from Wisent, and fetal bovine serum purchased from Biosera. CellTiter-Glo (chemiluminescence detection reagent for cell viability) reagent purchased from Promega. NCI-H358 cell line purchased from the Cell Bank of the Chinese Academy of Sciences. Nivo multi-label analyzer (PerkinElmer).

**Experimental methods:**

NCI-H358 cells were seeded in a white 96-well plate with 80 μL of cell suspension per well, which contained 4000 NCI-H358 cells. The cell plate was incubated overnight in a carbon dioxide incubator.

The compounds to be tested were diluted 3-fold with a row gun to the ninth concentration, that is, from 2 mM to 304 nM, and a double-duplication experiment was set up. 78 μL of culture medium was added to a middle plate, and then 2 μL of gradient diluted compound per well was transferred to the middle plate according to the corresponding position. After being thoroughly mixed, 20 μL per well was transferred to the cell plate. The concentration range of the compound transferred to the cell plate was 10 μM to 1.52 nM. The cell plate was placed in the carbon dioxide incubator for 5 days. Another cell plate was prepared, and the signal value was read as the maximum value (Max value in the equation below) on the day of administration to participate in data analysis. 25 μL of cell viability chemiluminescence detection reagent was added to each well of the cell plate, and incubated at room temperature for 10 minutes to stabilize the luminescence signal. A multi-label analyzer was used for reading.

25 μL of cell viability chemiluminescence detection reagent was added to each well of the cell plate, and incubated at room temperature for 10 minutes to stabilize the luminescence signal. A multi-label analyzer was used for reading.

**Data analysis:**

Using the equation **(Sample-Min)/(Max-Min)*100 %** to convert the raw data into inhibition rate, and the value of $IC_{50}$ might be obtained by curve fitting with four parameters (obtained by "log (inhibitor) vs. response--Variable slope" mode in GraphPad Prism). Table 1 provides the inhibitory activity of the compounds of the present disclosure on the

proliferation of NCI-H358 cells.

**Table 1**

| Sample | H358 $IC_{50}$ (nmol/L) |
|---|---|
| Compound 1A | 5.27 |
| Compound 1B | 16.2 |
| Compound 1C | 0.462 |
| Compound 1D | 0.917 |
| Compound 4 | 0.9 |
| Compound 5 | 4 |
| Compound 6 | 2 |

**Conclusion:**

The compounds of the present disclosure have a strong inhibitory effect on the proliferation of H358 cells.

**Experimental embodiment 2: Anti-proliferation experiment of MIA-PA-CA-2 cells**

**Experimental materials:**

DMEM culture medium, fetal bovine serum purchased from Biosera, and horse serum purchased from Gibco. CellTiter-Glo (chemiluminescence detection reagent for cell viability) reagent purchased from Promega. MIA-PA-CA-2 cell line purchased from Nanjing CoBioer Biotechnology Co., Ltd. EnVision multi-label analyzer (PerkinElmer).

**Experimental methods:**

MIA-PA-CA-2 cells were seeded in a white 96-well plate with 80 μL of cell suspension per well, which contained 1000 MIA-PA-CA-2 cells. The cell plate was incubated overnight in a carbon dioxide incubator.

The compounds to be tested were diluted 5-fold with a row gun to the eighth concentration, that is, from 2 mM to 26 nM, and a double-duplication experiment was set up. 78 μL of culture medium was added to a middle plate, and then 2 μL of gradient diluted compound per well was transferred to the middle plate according to the corresponding position. After being thoroughly mixed, 20 μL per well was transferred to the cell plate. The concentration range of the compound transferred to the cell plate was 10 μM to 0.13 nM. The cell plate was placed in the carbon dioxide incubator for 3 days. Another cell plate was prepared, and the signal value was read as the maximum value (Max value in the equation below) on the day of administration to participate in data analysis. 25 μL of cell viability chemiluminescence detection reagent was added to each well of the cell plate, and incubated at room temperature for 10 minutes to stabilize the luminescence signal. A multi-label analyzer was used for reading.

**Data analysis:**

Using the equation (Sample-Min)/(Max-Min)*100 % to convert the raw data into inhibition rate, and the value of $IC_{50}$ might be obtained by curve fitting with four parameters (obtained by "log (inhibitor)/response--Variable scope" mode in GraphPad Prism software). Table 2 provides the inhibitory activity of the compounds of the present disclosure on the proliferation of MIA-PA-CA-2 cells.

**Table 2**

| Sample | MIA-PA-CA-2 $IC_{50}$ (nmol/L) |
|---|---|
| Compound 1A | 46 |
| Compound 1B | 52 |
| Compound 1C | 7 |
| Compound 1D | 8 |

(continued)

| Sample | MIA-PA-CA-2 $IC_{50}$ (nmol/L) |
|---|---|
| Compound 4 | 1 |
| Compound 5 | 5 |
| Compound 6 | 4 |

**Conclusion:** The compounds of the present disclosure exhibit good inhibitory activity on the proliferation of MIA-PA-CA-2 cells.

**Experimental embodiment 3: Anti-tumor activity test on *in vivo* xenograft tumor model**

**Experimental objectives:**

Human non-small cell lung cancer H358 was used to investigate the tumor-inhibitory effect of the compound to be tested alone in an *in vivo* tumor model.

**Experimental methods:**

Female Balb/c nude mice were subcutaneously inoculated with the H358 human non-small cell lung cancer cell line, randomly grouped on the 6th day after inoculation according to tumor size and animal body weight, and administered as described below.

Vehicle control group: The administration was started in the afternoon of the grouping day, once a day, and the vehicle (10 % DMSO/60 % PEG400/30 % deionized water) was intragastrically administered at a dose of 0.1 mL/10 g body weight for 28 consecutive days.

Administration group: The administration was started in the afternoon of the grouping day, once a day, and 3 groups were intragastrically administered with compound 1D (10 % DMSO/60 % PEG400/ 30 % deionized water) at the doses of 1 mg/kg, 3 mg/kg and 10 mg/kg body weight, respectively, for 28 consecutive days.

During the experiment, the mice were weighed twice a week, and the tumor volume was measured (tumor volume was calculated according to the formula of length $\times$ width$^2$/2). The tumor inhibition rate TGI (%) was calculated according to the formula TGI (%) = [(1-mean tumor volume at the end of administration of a treatment group-mean tumor volume at the time of group administration of the treatment group)/(mean tumor volume at the end of treatment of vehicle control group-mean tumor volume at the time of group administration of vehicle control group)] $\times$ 100 %.

The relative tumor volume was calculated as RTV=TVn/TV1$\times$100 %

Where TV1 was the tumor volume on the day of group administration, and TVn was the tumor volume on the day of measurement.

Relative tumor proliferation rate (T/C %)=RTVt/RTVc$\times$100 %

Where RTVt was the mean relative tumor volume of the treatment group, and RTVc was the mean relative tumor volume of the vehicle control group.

**Experimental results:**

28 days after administration, the mean tumor volume of the vehicle control group was 669.16 mm$^3$, and the mean tumor volume of compound 1D at the doses of 1, 3 and 10 mg/kg were 341.36 mm$^3$, 199.4 mm$^3$ and 37.5 mm$^3$, respectively, with TGI of 58.78 %, 84.17 % and 113.19 %, with T/C of 51.35, 27.59 % and 5.76 %, respectively, and compound 1D could significantly inhibit the growth of tumor compared with the vehicle control group (P-values were 0.0629, 0.0029 and p<0.0001, respectively).

**Experimental conclusion:**

The compounds of the present disclosure have a good dose-dependent trend, can significantly inhibit the growth of tumor without obvious weight loss in mice, and are well tolerated.

**Experimental embodiment 4: Pharmacokinetic study of female BALB/c nude mice**

This experiment was a pharmacokinetic study of female BALB/c nude mice after oral administration of compound

1D. The experiment was divided into three groups, which were intragastrically administered with 1, 3 and 10 mg/kg of compound 1D, respectively. Animals in each group were divided into two batches, and plasma and tumor tissue samples were collected at different time points after administration. The collection time points of the plasma samples from each group of animals were: 0.083, 0.25, 0.5, 1, 2, 4, 6 and 8 hours, respectively, and the collection time points of tumor tissue samples were 1, 4 and 8 hours, respectively. At the corresponding sample collection time point after administration, plasma was collected, and tumor tissue was dissected and weighed. The plasma was stored at -80 °C, and the tumor tissue was stored in a cryopreservation tube and quickly frozen in liquid nitrogen. Cross-collected plasma samples were used to calculate the mean concentration of the corresponding time points in each group for the calculation of PK parameters.

**Experimental results:**

**Table 3 Pharmacokinetic parameters of plasma and tumor in female BALB/c nude mice after intragastric administration of compound 1D**

| Matrix | Plasma | | | Tumor | | |
|---|---|---|---|---|---|---|
| PK parameter | 1 mg/kg | 3 mg/kg | 10 mg/kg | 1 mg/kg | 3 mg/kg | 10 mg/kg |
| $C_{max}$ (nmol/L or nmol/kg) | 40.8 | 191 | 348 | NR | 136 | 203 |
| $T_{max}$ (h) | 0.250 | 0.500 | 0.250 | NR | 1.00 | 1.00 |
| $T_{1/2}$ (h) | 2.57 | 2.20 | 1.99 | NR | 1.28 | 2.19 |
| $T_{last}$ (h) | 8.00 | 8.00 | 8.00 | NR | 8.00 | 8.00 |
| $AUC_{0-last}$ (nmol/L.h or nmol/kg.h) | 54.4 | 287 | 840 | ND | 234 | 511 |
| AUC ratio | - | -- | -- | ND | 0.815 | 0.608 |
| "NR": lacking a terminal elimination phase or the exposure being too low;<br>"ND": not determined;<br>AUC ratio=tumor $AUC_{0-last}$/plasma $AUC_{0-last}$. | | | | | | |

**Table 4 Mean drug concentrations in plasma and tumor of female BALB/c nude mice at each time after intragastric administration of compound 1D**

| Compound | Compound 1D (nM) | | | | | |
|---|---|---|---|---|---|---|
| Dose | 1 mg/kg | | 3 mg/kg | | 10 mg/kg | |
| Time (h) | Plasma | Tumor | Plasma | Tumor | Plasma | Tumor |
| **1.00** | 22.5 | 10.4 | 157 | 136 | 285 | 203 |
| **4.00** | 3.58 | 2.04 | 11.5 | 9.78 | 54.8 | 37.9 |
| **8.00** | 1.22 | ND | 4.13 | 2.78 | 30.6 | 20.8 |
| "ND": not determined.<br>**Conclusion:** The compounds of the present disclosure have moderate metabolism speed in mice, high drug concentration in tumor tissue, and good pharmacokinetic properties. | | | | | | |

**Experimental embodiment 5: *In vivo* pharmacodynamic study of the compound on human colon cancer CO-04-0070 subcutaneous xenograft tumor model**

**Experimental objectives:**

To evaluate the *in vivo* efficacy of the compound in the human colon cancer CO-04-0070 subcutaneous xenograft tumor BALB/c nude mouse model.

**Tumor tissue inoculation and grouping:**

Each animal was inoculated with a CO-04-0070 FP7 tumor block with a volume of about 30 $mm^3$ in the right back position. When the mean tumor volume reached 151 $mm^3$, random grouping was used and the administration was started.

**Table 5 Grouping and administration scheme of experimental animals**

| Group | Drug | Dose (mg/kg) | Administration volume (μL/g)[2] | Route of administration | Frequency of administration |
|---|---|---|---|---|---|
| 1 | Vehicle control | - | 10 | PO | QD×3 weeks |
| 2 | Compound 1D | 3 | 10 | PO | QD×3 weeks |
| 3 | Compound 1D | 10 | 10 | PO | QD×3 weeks |
| 4 | Compound 1D | 30 | 10 | PO | QD×3 weeks |

**Tumor measurements and experimental indexes:**

Tumor diameters were measured with vernier calipers twice a week. The calculation formula of tumor volume was: $V = 0.5a \times b^2$, wherein *a* and *b* represented the long and short diameters of the tumor, respectively.

The anti-tumor efficacy of the compounds was evaluated by TGI (%) or relative tumor proliferation rate T/C (%). Relative tumor proliferation rate T/C(%) = $T_{RTV}$ / $C_{RTV}$ × 100 % ($T_{RTV}$: mean RTV of the treatment group; $C_{RTV}$: mean RTV of the negative control group). The relative tumor volume (RTV) was calculated according to the results of tumor measurement, and the calculation formula was RTV =Vt / $V_0$, wherein $V_0$ was the tumor volume measured at the time of group administration (that is, D0), Vt was the tumor volume at a certain measurement, and the data of $T_{RTV}$ and $C_{RTV}$ were taken on the same day. TGI (%), reflecting the tumor growth inhibition rate.

TGI (%) = [1-(mean tumor volume at the end of administration in a treatment group-mean tumor volume at the beginning of administration in the treatment group)/(mean tumor volume at the end of treatment in vehicle control group-mean tumor volume at the beginning of treatment in vehicle control group)] × 100 %.

After the experiment, the tumor weight would be detected, and the percentage of $T/C_{weight}$ would be calculated. $T_{weight}$ and $C_{weight}$ represented the tumor weight of the administration group and the vehicle control group, respectively.

**Experimental results:**

**Table 6 Tumor-inhibitory effect of compounds against human colon cancer CO-04-0070 xenograft tumor model**

| Group | Tumor volume ($mm^3$)[a] (Day 0) | Tumor volume ($mm^3$)[a] (Day 20) | RTV (Day 20) | TGI (%) (Day 20) | T/C (%) (Day 20) |
|---|---|---|---|---|---|
| Vehicle control group | 151 ± 13 | 1317 ± 73 | 9.18 ± 0.95 | -- | -- |
| Compound 1D (3 mg/kg) | 151 ± 14 | 788 ± 94 | 5.32 ± 0.56 | 45.4 | 58.0 |
| Compound 1D (10 mg/kg) | 151 ± 14 | 379 ± 86 | 2.43 ± 0.47 | 80.5 | 26.5 |

(continued)

| Group | Tumor volume ($mm^3$)[a] (Day 0) | Tumor volume ($mm^3$)[a] (Day 20) | RTV (Day 20) | TGI (%) (Day 20) | T/C (%) (Day 20) |
|---|---|---|---|---|---|
| Compound 1D (30 mg/kg) | 151 ± 13 | 152 ± 21 | 1.02 ± 0.11 | 99.9 | 11.1 |

Note: a. mean ± SEM, n = 8.

**Table 7 Tumor weight in each group**

| Group | Tumor weight (g)[a] (Day 20) | $T/C_{weight}$[b] (%) | *p* value[c] |
|---|---|---|---|
| Vehicle control group | 1.350 ± 0.068 | -- | -- |
| Compound 1D (3 mg/kg) | 0.832 ± 0.102 | 61.6 | 0.012 |
| Compound 1D (10 mg/kg) | 0.406 ± 0.095 | 30.1 | <0.001 |
| Compound 1D (30 mg/kg) | 0.162 ± 0.023 | 12.0 | <0.001 |

Note: a. mean ± SEM, n = 8.
b. Tumor growth inhibition was calculated by $T/C_{weight} = TW_{treatment} / TW_{vehicle}$.
c. The *p* value was obtained by analyzing the tumor weight using one-way ANOVA and the vehicle treatment group. F value was significantly different ($p < 0.01$), and the Games-Howell method was used for analysis.

Changes in body weight: There was no significant weight loss in all administration groups in this model

**Conclusion:** The compounds of the present disclosure have significant inhibitory effect on tumor growth and shows a certain dose dependence.

**Experimental embodiment 6: Experiment on the protein levels of ERK and p-ERK in tumor tissue**

In this experiment, the protein levels of ERK and p-ERK in tumor tissues of different administration groups after administration of compound 1D (30 mg/kg) for 6 hours in human colon cancer CO-04-0070 subcutaneous xenograft tumor model were analyzed by immunoblotting.

**Experimental materials:**

**1) Reagents**

| | Reagent | Supplier |
|---|---|---|
| 1 | RIPA cell lysate | Sigma |
| 2 | Phosphatase inhibitor | Sigma |
| 3 | Protease inhibitor | Roche |
| 4 | BCA protein quantification kit | Thermo Scientific |
| 5 | LDS loading buffer (4x) | Thermo Scientific |
| 6 | Sample reducing agent (10x) | Thermo Scientific |
| 7 | NuPAGE® MES SDS Running Buffer (20x) | Thermo Scientific |
| 8 | 4-12 % Bis-Tris Midi Protein Gels 26well | Invitrogen |
| 9 | PageRuler™ Prestained Protein Ladder | Thermo Scientific |
| 10 | 20x TBS | Bio-Serve |
| 11 | Tween 20 | Sigma |

(continued)

| | Reagent | Supplier |
|---|---|---|
| 12 | iBlot® 2 transmembrane kit | Thermo Scientific |
| 13 | Bovine serum albumin | BBI life sciences & services |
| 14 | SuperSignal™ West Femto Maximum | Thermo Scientific |
| | Senxitivity Substrate | |
| 15 | Skim milk powder | Brightdairy |

**2) Instruments**

| | Instrument | Supplier |
|---|---|---|
| 1 | Tissuelyser LT | Qiagen |
| 2 | Digital display electrothermal incubator | Shanghai Boxun Industry & Commerce Co.,Ltd, Medical Equipment Factory |
| 3 | SpectraMax iD5 | Molecular Devices |
| 4 | Dry thermostat | Haimen City Qilin Medical Instrument Factory |
| 5 | XCell SureLock® electrophoresis cell | Thermo Scientific |
| 6 | PowerEase® 500 power supplies | Thermo Scientific |
| 7 | iBlot®2 transmembrane device | Thermo Scientific |
| 8 | Tanon5200 Multi | Tanon |

**3) Antibodies**

| | Antibody | Supplier |
|---|---|---|
| 1 | β-Actin Antibody | Cell Signaling Technology |
| 2 | p44/42 MAPK (Erk1/2) (137F5) Rabbit mAb | Cell Signaling Technology |
| 3 | Phospho-p44/42 MAPK (Erk1/2) (Thr202/Tyr204) (197G2) Rabbit mAb | Cell Signaling Technology |
| 4 | Goat anti- Rabbit IgG-HRP (anti Rabbit secondary antibody) | Thermo Shift |

**Experimental methods:**

1) Protein extraction and quantification

Quick-frozen tissue samples were removed from -80 °C refrigerator, operated on dry ice. Part of the tissues (about 50-100 mg) was cut, put into a 2 mL centrifuge tube with steel beads, and 500 μL of cell lysate RIPA (1 % protease inhibitor and phosphatase inhibitor had been freshly added) was added thereto. Tissuelyser LT disrupted the tissue for 5 minutes using the highest frequency. The tissue lysate was lysed on ice for 30 minutes, centrifuged at 12,000 rpm at 4 °C for 10 minutes, and the supernatant was transferred to a new 1.5 mL centrifuge tube. Protein quantification was performed with a BCA quantification kit. According to the quantification results, the protein sample for loading was configured, and the sample protein concentration was unified to 2 μg/μL, and LDS loading buffer (4 times) and sample reducing agent (10 times) were added thereto. The samples were heated at a constant temperature of 100 °C for 10 minutes. Western blot, or denatured samples were stored in the -80 °C refrigerator.

2) Immunoblotting

The sample for loading was thawed. Sample loading: 10 μL of sample was loaded per well in SDS-PAGE gel. Electrophoresis: 80 volts, 30 minutes, followed by 120 volts, 90 minutes. Transmembrane: Using an iBlot2 transmembrane kit and a transmembrane device for transmembrane, and a P3 program was run for 7 minutes. After the transmembrane was completed, the membrane was cut according to the molecular weight of the protein to be detected. Sealing: The membrane was sealed in a sealing liquid (5 % skim milk configured with 1 × TBST) at room temperature and shaken for 1 hour. Incubation of primary antibody: Primary antibody at an appropriate dilution (1:1000) (diluted with 5 % skim milk or bovine serum albumin configured with 1 × TBST) was added, overnight at 4 °C with slow shaking. The membrane was washed with 1 × TBST, 3 times for 10 minutes each, at room temperature with shaking. Incubation of secondary antibody: Secondary antibody at an appropriate dilution (1:10000) was added at room temperature with slow shaking for 1 hour. The membrane was washed with 1 × TBST, 3 times for 10 minutes each, at room temperature with shaking. Chemiluminescence: A HRP substrate from the West Femto supersensitive chemiluminescence kit was added to the membrane. Chemiluminescence was detected and photographed on a Tanon 5200 multi machine.

3) Expression quantification

The relative quantification of the density intensity of the chemiluminescence bands in immunoblotting was performed using ImageJ and other related software.

**Experimental results:** See Figs. 1 and 2.

In Fig. 2 "**" indicates a two-tailed T-test with *p*-value < 0.01. "Normalized fold change" is the normalized change multiple.

Conclusion: The expression level of p-ERK protein in the tumor tissue of the compound treatment group of the present disclosure is significantly decreased.

## Claims

1. A compound represented by formula (III) or a pharmaceutically acceptable salt thereof,

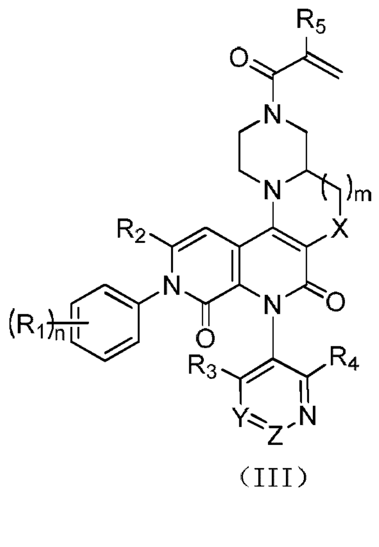

(III)

wherein,

m is selected from 0, 1 and 2;
n is selected from 0, 1, 2, 3 and 4;
X is selected from NH and O;
Y is selected from CH and N;
Z is selected from CH and N;
$R_1$ is each independently H, F, Cl, Br, I, OH, $NH_2$ and $C_{1-3}$ alkyl, and the $C_{1-3}$ alkyl is optionally substituted by 1, 2, or 3 $R_a$;

$R_2$ is selected from H, F, Cl, Br, I and $C_{1-3}$ alkyl, and the $C_{1-3}$ alkyl is optionally substituted by 1, 2, or 3 $R_b$;
$R_3$ is selected from H, F, Cl, Br, I and $C_{1-6}$ alkyl, and the $C_{1-6}$ alkyl is optionally substituted by 1, 2, or 3 $R_c$;
$R_4$ is selected from H, F, Cl, Br, I and $C_{1-6}$ alkyl, and the $C_{1-6}$ alkyl is optionally substituted by 1, 2, or 3 $R_c$;
$R_5$ is selected from H, F, Cl, Br and I;
$R_a$, $R_b$ and $R_c$ are each independently selected from H, F, Cl, Br and I.

2. The compound or the pharmaceutically acceptable salt thereof according to claim 1, wherein, the compound has a structure of formula (II):

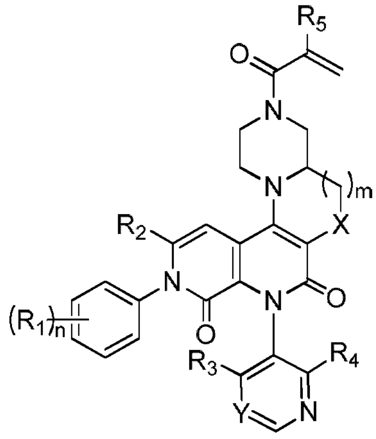

(II) ,

$R_1$, $R_2$, $R_3$, $R_4$, $R_5$, X, Y, m and n are as defined in claim 1.

3. The compound or the pharmaceutically acceptable salt thereof according to claim 1 or 2, wherein, $R_1$ is each independently selected from F, Cl, $NH_2$ and OH.

4. The compound or the pharmaceutically acceptable salt thereof according to claim 1 or 2, wherein, $R_2$ is selected from $CH_3$, and the $CH_3$ is optionally substituted by 1, 2 or 3 $R_b$.

5. The compound or the pharmaceutically acceptable salt thereof according to claim 4, wherein, $R_2$ is selected from $CF_3$.

6. The compound or the pharmaceutically acceptable salt thereof according to claim 1 or 2, wherein, $R_3$ is selected from $CH_3$, $CH_2CH_3$ and

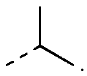

7. The compound or the pharmaceutically acceptable salt thereof according to claim 1 or 2, wherein, $R_4$ is selected from $CH_3$, $CH_2CH_3$ and

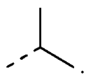

8. The compound or the pharmaceutically acceptable salt thereof according to claim 1 or 2, wherein, $R_5$ is selected from Hand F.

9. The compound or the pharmaceutically acceptable salt thereof according to claim 1 or 2, wherein, the structural moiety

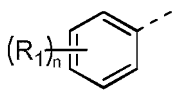

is selected from

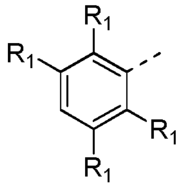

.

**10.** The compound or the pharmaceutically acceptable salt thereof according to claim 9, wherein, the structural moiety

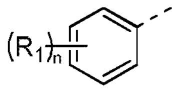

is selected from

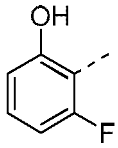

and

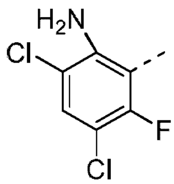

.

**11.** The compound or the pharmaceutically acceptable salt thereof according to claim 1, wherein, the structural moiety

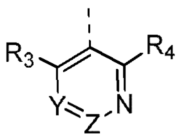

is selected from

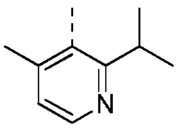

,

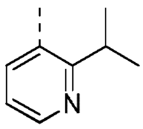

,

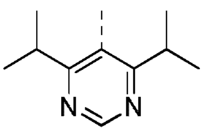

,

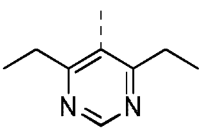

and

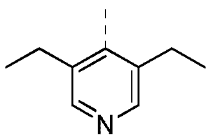

.

**12.** The compound or the pharmaceutically acceptable salt thereof according to claim 2, wherein, the structural moiety

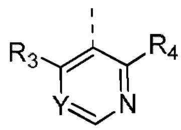

is selected from

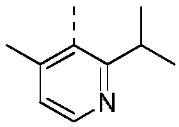 , 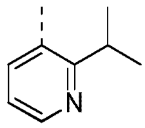 ,

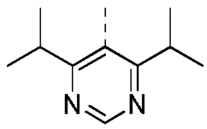 and 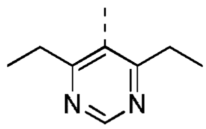 .

13. The compound or the pharmaceutically acceptable salt thereof according to any one of claims 1 to 9, wherein, the compound is selected from

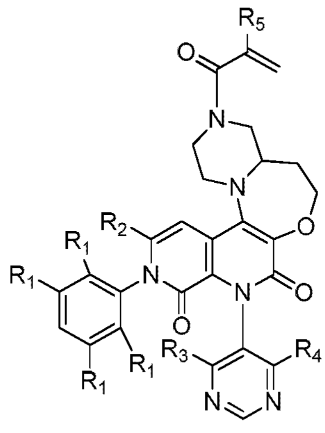
(II-2) ,

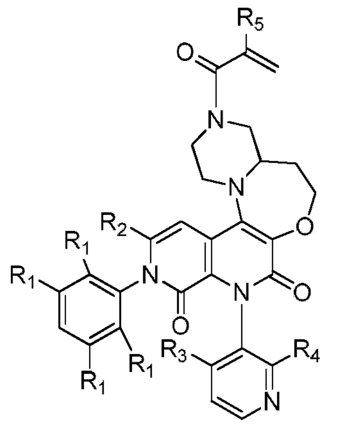
(II-3) and

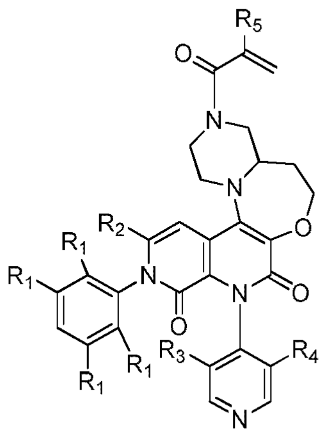
(III-1) ,

wherein, $R_1$, $R_2$, $R_3$, $R_4$ and $R_5$ are as defined in any one of claims 1 to 9.

14. A compound represented by the following formula or a pharmaceutically acceptable salt thereof, selected from

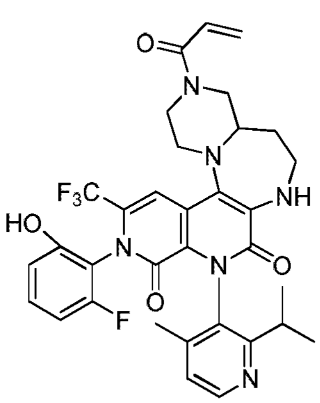 , 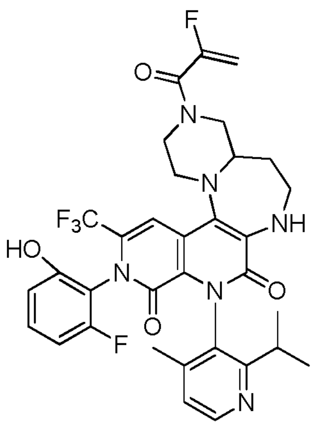 , 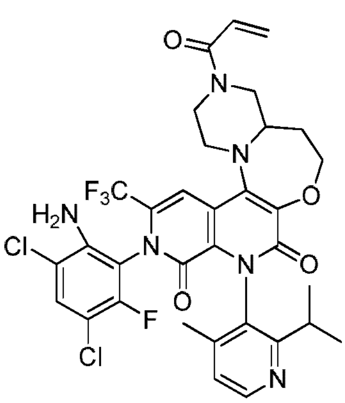 ,

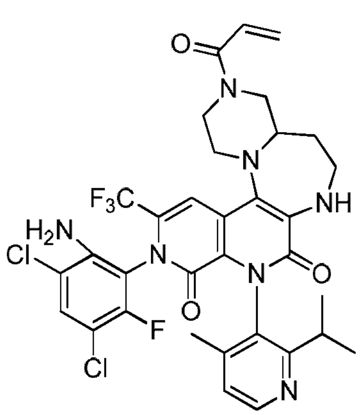,
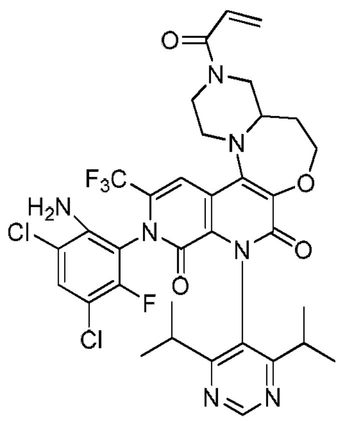,
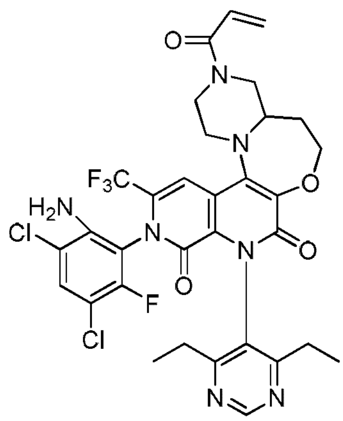

and

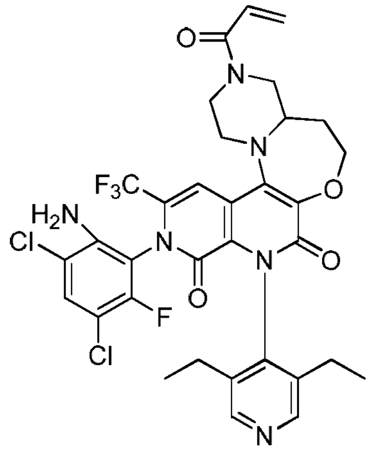.

**15.** The compound or the pharmaceutically acceptable salt thereof according to claim 14, selected from

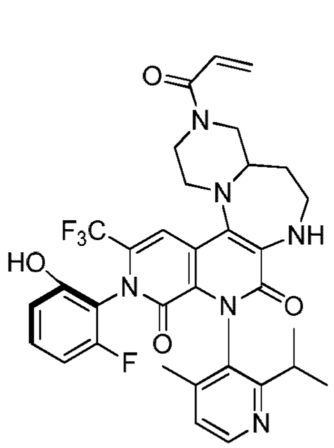,
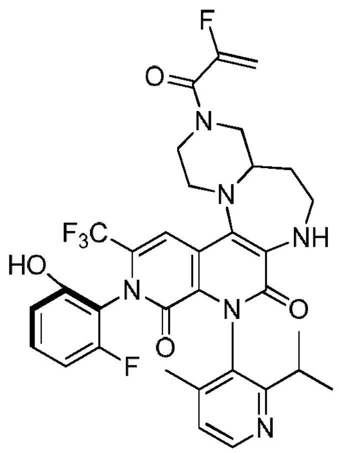,
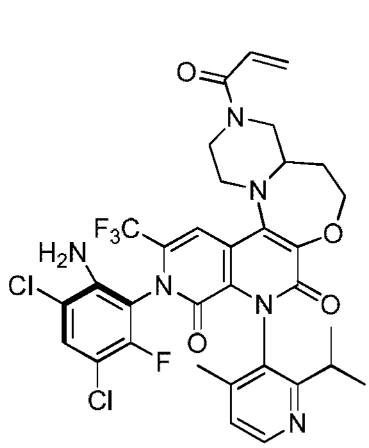,

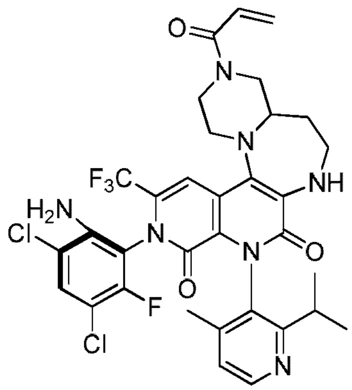
,
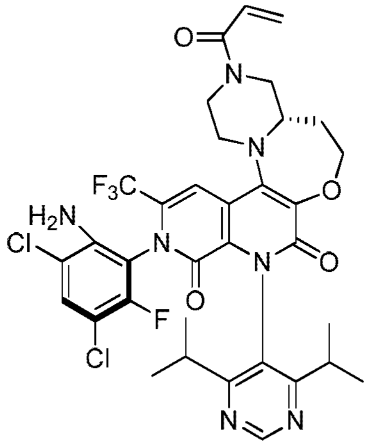
,
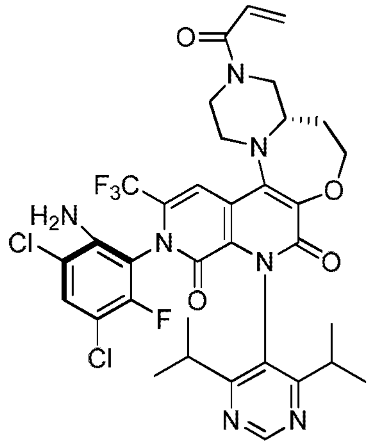
,
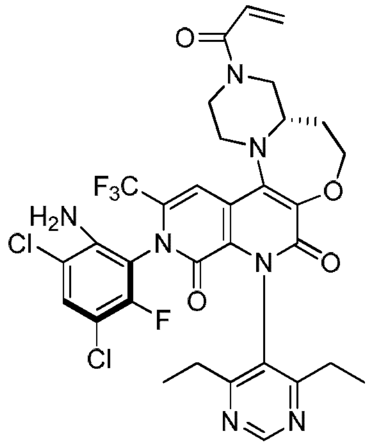
,
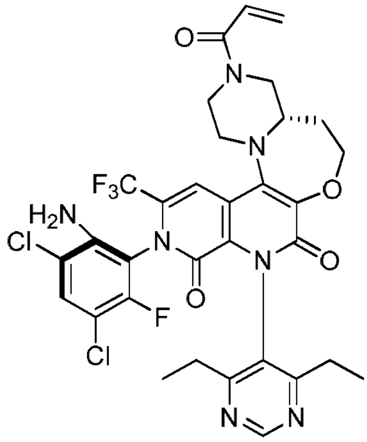
,
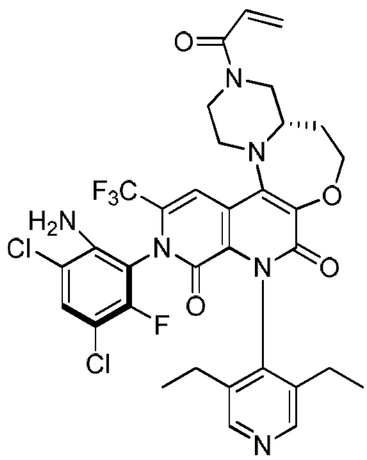

and

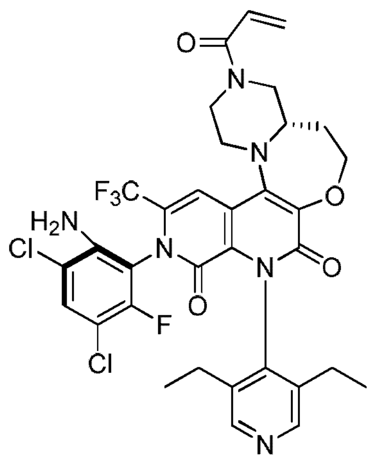
.

**16.** A pharmaceutical composition, comprising a therapeutically effective amount of the compound or the pharmaceutically acceptable salt thereof according to any one of claims 1 to 15 as an active ingredient and a pharmaceutically acceptable carrier.

**17.** Use of the compound or the pharmaceutically acceptable salt thereof according to any one of claims 1 to 15 or the pharmaceutical composition according to claim 16 in the manufacture of a KRAS G12C mutant protein inhibitor.

**18.** Use of the compound or the pharmaceutically acceptable salt thereof according to any one of claims 1 to 15 or the

pharmaceutical composition according to claim 16 in the manufacture of a medicament for the treatment of cancer.

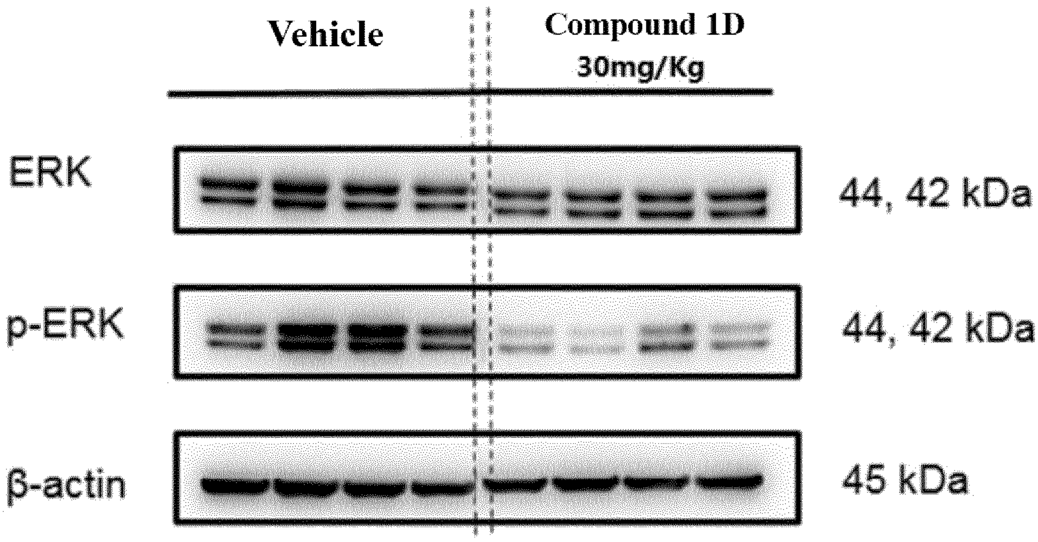
Vehicle
Compound 1D
30mg/Kg
ERK
44, 42 kDa
p-ERK
44, 42 kDa
β-actin
45 kDa

Fig. 1

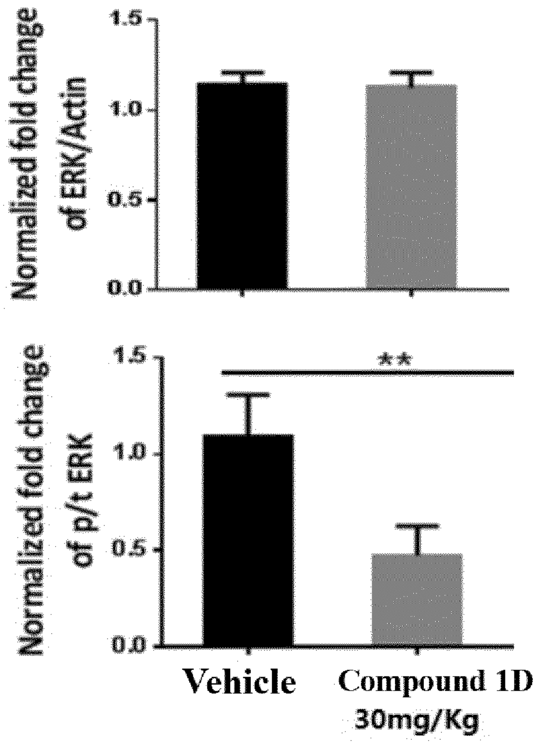
Normalized fold change of ERK/Actin
1.5
1.0
0.5
0.0
Normalized fold change of p/t ERK
**
1.5
1.0
0.5
0.0
Vehicle
Compound 1D
30mg/Kg

Fig. 2

## INTERNATIONAL SEARCH REPORT

International application No.
**PCT/CN2021/085733**

**A. CLASSIFICATION OF SUBJECT MATTER**

C07D 403/14(2006.01)i; C07D 413/14(2006.01)i; C07D 498/22(2006.01)i; A61K 31/551(2006.01)i; A61K 31/553(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

DWPI; SIPOABS; CNKI; CNABS; STNext; Web of Science: 南京明德新药研发, KRAS, G12C, 癌症, 肿瘤, tumor, cancer, structural formula search

**C. DOCUMENTS CONSIDERED TO BE RELEVANT**

| Category* | Citation of document, with indication, where appropriate, of the relevant passages | Relevant to claim No. |
|---|---|---|
| PX | WO 2020239123 A1 (SHANGHAI HANSOH BIOMEDICAL CO., LTD. et al.) 03 December 2020 (2020-12-03)<br>description abstract, claims 1-21, compounds iii-16, iii-17, iii-18, compounds 30-36 | 1-18 |
| Y | WO 2019213516 A1 (AMGEN INC.) 07 November 2019 (2019-11-07)<br>description abstract, claims 1-28, embodiments 9-11 | 1-18 |
| Y | US 2019177338 A1 (ASTRAZENECA AB.) 13 June 2019 (2019-06-13)<br>the abstract, and claims 1-25 | 1-18 |
| A | WO 2018206539 A1 (ASTRAZENECA AB.) 15 November 2018 (2018-11-15)<br>abstract, and claims 1-16 | 1-18 |
| A | WO 2018217651 A1 (AMGEN INC.) 29 November 2018 (2018-11-29)<br>abstract and claims 1-32 | 1-18 |
| PA | WO 2021052499 A1 (SHANGHAI JEMINCARE PHARMACEUTICALS CO., LTD. et al.) 25 March 2021 (2021-03-25)<br>abstract, and claims 1-27 | 1-18 |
| A | CN 110366550 A (AMGEN INC.) 22 October 2019 (2019-10-22)<br>description abstract, claims 1-93 | 1-18 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **16 June 2021** | **07 July 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China**<br>Facsimile No. **(86-10)62019451** | Authorized officer<br><br>Telephone No. |

Form PCT/ISA/210 (second sheet) (January 2015)

INTERNATIONAL SEARCH REPORT

International application No.
PCT/CN2021/085733

C. DOCUMENTS CONSIDERED TO BE RELEVANT

| Category* | Citation of document, with indication, where appropriate, of the relevant passages | Relevant to claim No. |
|---|---|---|
| A | WO 2019051291 A1 (AMGEN INC.) 14 March 2019 (2019-03-14)<br>description abstract, claims 1-81 | 1-18 |
| A | CN 104844573 A (CHINA PHARMACEUTICAL UNIVERSITY) 19 August 2015 (2015-08-19)<br>abstract, and claims 1-9 | 1-18 |

Form PCT/ISA/210 (second sheet) (January 2015)

**INTERNATIONAL SEARCH REPORT**
**Information on patent family members**

International application No.
**PCT/CN2021/085733**

| Patent document cited in search report | Publication date (day/month/year) | Patent family member(s) | Publication date (day/month/year) |
|---|---|---|---|
| WO 2020239123 A1 | 03 December 2020 | None | |
| WO 2019213516 A1 | 07 November 2019 | EP 3788038 A1 | 10 March 2021 |
| | | CA 3098574 A1 | 07 November 2019 |
| | | AU 2019262589 A1 | 29 October 2020 |
| | | US 2019343838 A1 | 14 November 2019 |
| US 2019177338 A1 | 13 June 2019 | WO 2019110751 A1 | 13 June 2019 |
| | | TW 201938561 A | 01 October 2019 |
| | | US 10597405 B2 | 24 March 2020 |
| | | UY 38001 A | 31 May 2019 |
| WO 2018206539 A1 | 15 November 2018 | EP 3621968 A1 | 18 March 2020 |
| | | CA 3061650 A1 | 15 November 2018 |
| | | JP 2020519589 A | 02 July 2020 |
| | | TW 201906848 A | 16 February 2019 |
| | | CN 110603258 A | 20 December 2019 |
| | | AR 111776 A1 | 21 August 2019 |
| | | US 2020109153 A1 | 09 April 2020 |
| WO 2018217651 A1 | 29 November 2018 | JP 6785819 B2 | 18 November 2020 |
| | | KR 20200010384 A | 30 January 2020 |
| | | US 2021009577 A1 | 14 January 2021 |
| | | CA 3063469 A1 | 29 November 2018 |
| | | BR 112019024525 A2 | 09 June 2020 |
| | | CR 20190534 A | 20 March 2020 |
| | | US 2020055845 A1 | 20 February 2020 |
| | | JP 2021020948 A | 18 February 2021 |
| | | AU 2018273356 A1 | 21 November 2019 |
| | | EA 201992781 A1 | 01 April 2020 |
| | | MX 2019013858 A | 20 January 2020 |
| | | CO 2019013010 A2 | 01 April 2020 |
| | | PE 20200733 A1 | 23 July 2020 |
| | | EP 3630761 A1 | 08 April 2020 |
| | | US 2018334454 A1 | 22 November 2018 |
| | | CL 2019003394 A1 | 13 April 2020 |
| | | AR 111882 A1 | 28 August 2019 |
| | | CN 110997668 A | 10 April 2020 |
| | | CL 2020002405 A1 | 28 December 2020 |
| | | JP 2019031476 A | 28 February 2019 |
| | | UY 37744 A | 02 January 2019 |
| | | PH 12019502579 A1 | 13 July 2020 |
| | | TW 201906821 A | 16 February 2019 |
| | | US 10519146 B2 | 31 December 2019 |
| | | SG 10201913195R A | 27 February 2020 |
| WO 2021052499 A1 | 25 March 2021 | None | |
| CN 110366550 A | 22 October 2019 | AU 2017379074 A1 | 18 July 2019 |
| | | SG 11201906223 T A | 27 August 2019 |
| | | CL 2020000784 A1 | 28 August 2020 |
| | | IL 267495 D0 | 29 August 2019 |
| | | EP 3558955 A2 | 30 October 2019 |
| | | SG 10201913491 P A | 30 March 2020 |
| | | PE 20191207 A1 | 10 September 2019 |

Form PCT/ISA/210 (patent family annex) (January 2015)

**INTERNATIONAL SEARCH REPORT**
**Information on patent family members**

International application No.
**PCT/CN2021/085733**

| Patent document cited in search report | Publication date (day/month/year) | Patent family member(s) | Publication date (day/month/year) |
|---|---|---|---|
| | | CL 2019001762 A1 | 18 October 2019 |
| | | US 2020069657 A1 | 05 March 2020 |
| | | JP 2020504738 A | 13 February 2020 |
| | | CR 20190338 A | 09 September 2019 |
| | | US 2018177767 A1 | 28 June 2018 |
| | | KR 20190097201 A | 20 August 2019 |
| | | PH 12019501670 A1 | 04 November 2019 |
| | | US 10532042 B2 | 14 January 2020 |
| | | CA 3048217 A1 | 28 June 2018 |
| | | WO 2018119183 A2 | 28 June 2018 |
| | | WO 2018119183 A3 | 23 August 2018 |
| | | MX 2019007643 A | 09 September 2019 |
| | | BR 112019012976 A2 | 31 December 2019 |
| | | ZA 201904548 B | 25 March 2020 |
| | | CO 2019006598 A2 | 28 June 2019 |
| | | EA 201991528 A1 | 16 January 2020 |
| WO 2019051291 A1 | 14 March 2019 | US 10640504 B2 | 05 May 2020 |
| | | AR 112797 A1 | 11 December 2019 |
| | | EP 3679040 A1 | 15 July 2020 |
| | | UY 37870 A | 29 March 2019 |
| | | CN 111051306 A | 21 April 2020 |
| | | CA 3075046 A1 | 14 March 2019 |
| | | IL 272512 D0 | 31 March 2020 |
| | | US 2020207766 A1 | 02 July 2020 |
| | | SG 11202001499 W A | 30 March 2020 |
| | | AU 2018329920 A1 | 27 February 2020 |
| | | JP 2020533277 A | 19 November 2020 |
| | | TW 201922739 A | 16 June 2019 |
| | | US 2019077801 A1 | 14 March 2019 |
| CN 104844573 A | 19 August 2015 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202010260612X
- CN 202010568775
- CN 202011616152